# EUROPEAN PATENT APPLICATION

(11) **EP 2 626 356 A1**
(43) Date of publication of application: **14.08.2013**
(21) Application number: 12154531.3
(22) Date of filing: 08.02.2012
(51) Int. Cl.: C07D 403/06, A61K 31/506, A61P 31/04

(54) **Thiamine analogues and their use as antibiotics**

(71) Applicant: Rheinische Friedrich-Wilhelms-Universität Bonn, 53113 Bonn (DE)
(72) Inventor: Mayer, Günter, 53123 Bonn (DE); Lünse, Christina Elsbeth, 53111 Bonn (DE); Suckling, Colin, Bearsden, Glasgow G61 3AF (GB); Scott, Fraser, Houston, Johnstone, Glasgow PA6 7EX (GB)
(74) Representative: Michalski Hüttermann & Partner Patentanwälte

(57) **Abstract**

The present invention relates to compounds according to general formula (I), pharmaceutical compositions comprising compounds according to general formula (I) and the use of the compounds for the treatment of a bacterial infection, particularly for use as an antibiotic.

## Description

The present invention relates to compounds for use in the treatment of a bacterial infection, pharmaceutical compositions, and methods for treating bacterial infection.

Although most bacteria are harmless or beneficial, several bacteria are pathogenic and cause bacterial infections. Bacterial infections can be treated with antibiotics. A number of different types of antibiotics of highly different chemical structure have been identified, but since the discovery of antibiotic substances and their use against microbes, bacteria have evolved to defend themselves by acquiring resistances and the efficacy of antibiotics is hampered by the spreading of bacterial resistances. Especially the increasing appearance of multi-resistant pathogenic strains seriously threatens the ability to control many microbial pathogens today. Antibiotic resistance creates a permanent need for new antibacterial substances.

New antibacterial targets are constituted by the lately discovered riboswitches. Riboswitches regulate gene expression by binding to a metabolite and are involved in the regulation of up to 2-4% of all bacterial genes. Riboswitches are RNA elements mostly found in the 5'-untranslated region (5'UTR) of bacterial mRNA, consisting of a metabolite-binding aptamer domain and an expression domain. Upon metabolite binding, a change in secondary structure leads to inhibition of transcription or translation initiation.

In H. influenza and other pathogenic bacteria the TPP (thiamine pyrophosphate) riboswitch also known as the thi-box riboswitch has been shown to be involved in the regulation of essential genes. The activation of the thi-box riboswitch is triggered by binding to the metabolite thiamine pyrophosphate (TPP). Further, the antibacterially active thiamine analog pyrithiamine is mediated by interaction with the TPP or thi-box riboswitches. Although chemically highly different structures with UD 40345 / SAM:AL antibacterial activity have been identified, it is nearly impossible to predict which compound may exhibit antibiotic characteristics.

Therefore, the object underlying the present invention was to provide compounds that are able to modulate thi-box riboswitch activity.

The problem is solved by a compound according to general formula (I) as given as follows and/or racemates, enantiomers, diastereomers, solvates, hydrates, and pharmaceutically acceptable salts and/or esters thereof: 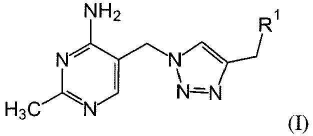 wherein:
- R¹: is selected from the group comprising -OH, -CH₂-NH₂, -(CH₂)₂-OH, -(CH₂)₃-OH, -CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given as follows:

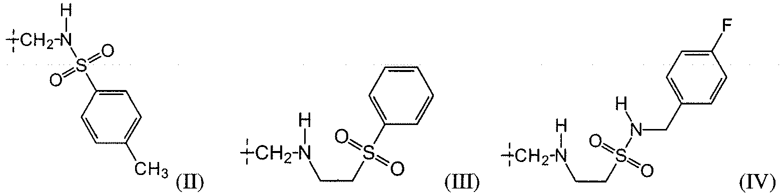

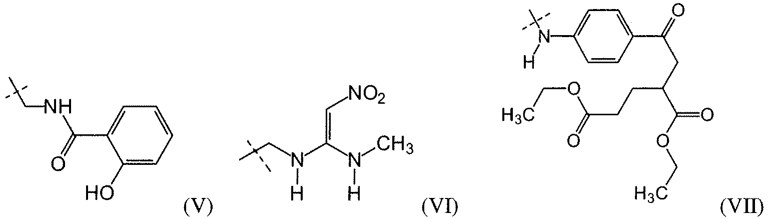

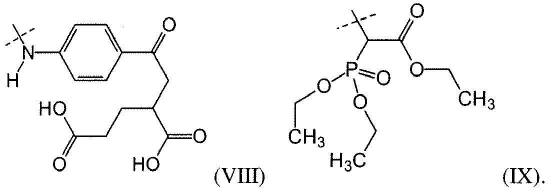

Furthermore, the invention relates to a pharmaceutical composition comprising such compounds as an active ingredient, the use of a compound according to the invention in the treatment of a bacterial infection and a method for preparing such compounds.

Surprisingly it was found that the compounds according to the invention can exhibit an affinity and are able to activate the thi-box riboswitch. Beneficially, the compounds according to the invention can be useful as antibacterial drugs in the treatment of bacterial infections.

In preferred embodiments, the compound is selected from the triazole thiamines according to formulas (2) to (17) as indicated below and/or racemates, enantiomers, diastereomers, solvates, hydrates, and pharmaceutically acceptable salts and/or esters thereof:

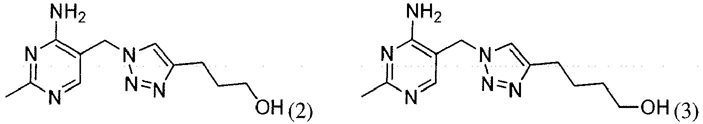

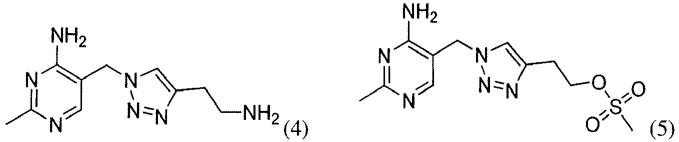

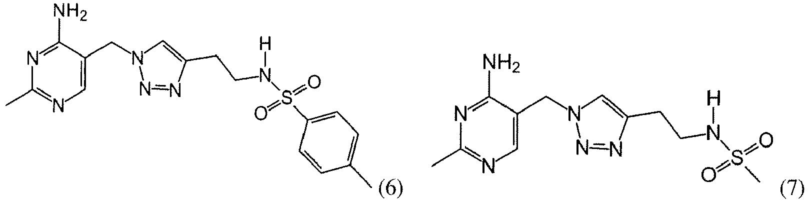

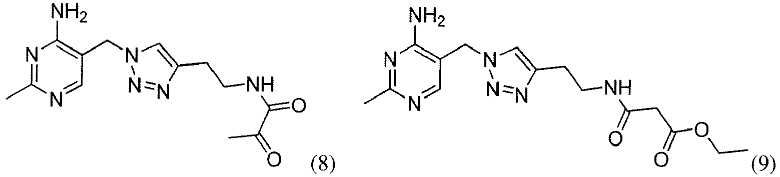

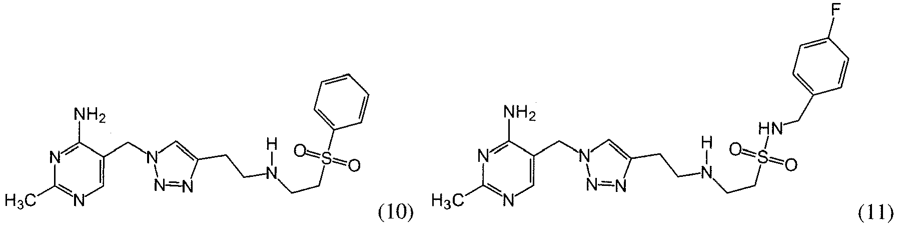

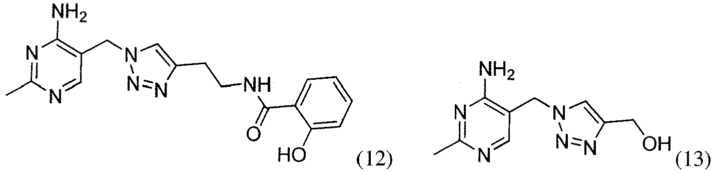

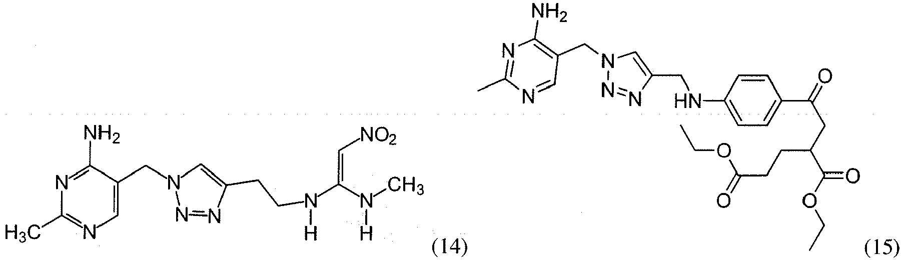

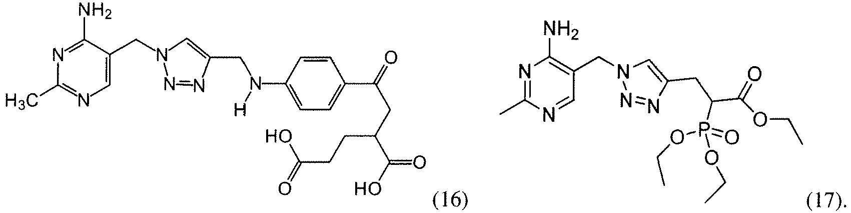

The compounds according to formulas (2) to (17) are denoted:
- 3-(1-((4-amino-2-methylpyrimidin-5-yl)methyl)-1H-1,2,3-triazol-4-yl)propan-1-ol,
- 4-(1-((4-amino-2-methylpyrimidin-5-yl)methyl)-1H-1,2,3-triazol-4-yl)butan-1-ol,
- 5-((4-(2-aminoethyl)-1H-1,2,3-triazol-1-yl)methyl)-2-methylpyrimidin-4-amine,
- 2-(1-((4-amino-2-methylpyrimidin-5-yl)methyl)-1H-1,2,3-triazol-4-yl)ethyl methanesulfonate,
- N-(2-(1-((4-amino-2-methylpyrimidin-5-yl)methyl)-1H-1,2,3-triazol-4-yl)ethyl)-4-methylbenzenesulfonamide,
- N-(2-(1-((4-amino-2-methylpyrimidin-5-yl)methyl)-1H-1,2,3-triazol-4-yl)ethyl)methanesulfonamide,
- N-(2-(1-((4-amino-2-methylpyrimidin-5-yl)methyl)-1H-1,2,3-triazol-4-yl)ethyl)-2-oxopropanamide,
- ethyl 3-((2-(1-((4-amino-2-methylpyrimidin-5-yl)methyl)-1H-1,2,3-triazol-4-yl)ethyl)amino)-3-oxopropanoate,
- 2-(1-((4-amino-2-methylpyrimidin-5-yl)methyl)-1H-1,2,3-triazol-4-yl)-N-(2-(phenylsulfonyl)ethyl)ethanamine,
- 2-(1-((4-amino-2-methylpyrimidin-5-yl)methyl)-1H-1,2,3-triazol-4-yl)-N-(2-(N-(4-fluorobenzyl)sulfamoyl)ethyl)ethanamine,
- N-(2-(1-((4-amino-2-methylpyrimidin-5-yl)methyl)-1H-1,2,3-triazol-4-yl)ethyl)-2-hydroxybenzamide,
- (1-((4-amino-2-methylpyrimidin-5-yl)methyl)-1H-1,2,3-triazol-4-yl)methanol,
- (E)-4-amino-2-methyl-5-((4-(2-((1-(methylamino)-2-nitrovinyl)amino)ethyl)-1H-1,2,3-triazol-1-yl)methyl)pyrimidine,
- diethyl 2-(2-(4-(((1-((4-amino-2-methylpyrimidin-5-yl)methyl)-1H-1,2,3-triazol-4-yl)methyl)amino)phenyl)-2-oxoethyl)pentanedioate,
- 4-amino-5-((4-(((4-(3,5-dicarboxypentanoyl)phenyl)amino)methyl)-1H-1,2,3-triazol-1-yl)methyl)-2-methylpyrimidine, and
- ethyl 3-(1-((4-amino-2-methylpyrimidin-5-yl)methyl)-1H-1,2,3-triazol-4-yl)-2-(diethoxyphosphoryl)propanoate,
respectively, according to the IUPAC nomenclature.

Unless specifically stated otherwise, compounds, groups or substituents denoted with Arabic numerals and such compounds, groups or substituents denoted with Roman numerals differ from each other, that is, compounds, groups or substituents are different compounds, groups or substituents.

The compounds described herein contain one or more asymmetric centres and may thus give rise to stereo isomers (configurational isomers). The present invention includes all such possible stereo isomers as well as their mixtures, and pharmaceutically acceptable salts thereof.

In a preferred embodiment, the compound has the general formula (X) as given as follows and/or racemates, enantiomers, diastereomers, solvates, hydrates, and pharmaceutically acceptable salts and/or esters thereof: 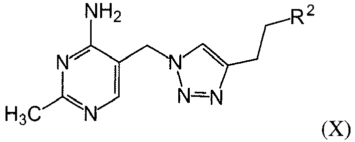 wherein:
- R²: is selected from the group comprising -NH₂, -CH₂-OH, -(CH₂)₂-OH, -O-S(O)₂CH₃, -NH-S(O)₂CH₃, -NH-C(O)-C(O)-CH₃, -NH-C(O)-CH₂-C(O)-O-CH₂-CH₃, and/or sulfones according to formulas (XI), (XII), and (XIII) as given as follows

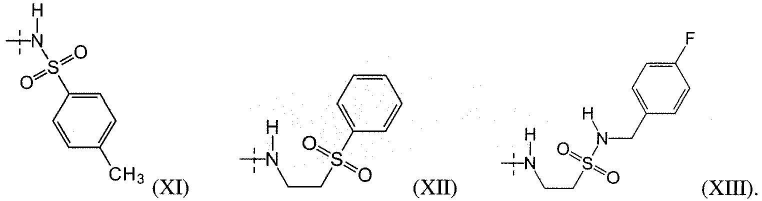

Advantageously, compounds according to the general formula (X) can exhibit a good affinity to the thi-box riboswitch and can be particularly useful as an antibacterial substance in the treatment of bacterial infection.

Preferably, the compound is selected from the triazole thiamines according to formulas (2) to (12) and (13) and/or racemates, enantiomers, diastereomers, solvates, hydrates, and pharmaceutically acceptable salts and/or esters thereof. The compounds according to formulas (2) to (12) and (13) are able to activate the thi-box riboswitch and represent promising antibacterial compounds.

Preferred acceptable salts of the compounds are chlorides and trifluoroacetates. Chlorides and trifluoroacetates dissolve easily, especially in water, and are generally considered non-toxic. For example, the compounds according to the formulas (6), (7), (10), (11), (14), and (16) are also usable as their trifluoroacetates, accordingly denoted:
- N-(2-(1-((4-amino-2-methylpyrimidin-5-yl)methyl)-1H-1,2,3-triazol-4-yl)ethyl)-4-methylbenzenesulfonamide 2,2,2-trifluoroacetate,
- N-(2-(1-((4-amino-2-methylpyrimidin-5-yl)methyl)-1H-1,2,3-triazol-4-yl)ethyl)methanesulfonamide 2,2,2-trifluoroacetate,
- 2-(1-((4-amino-2-methylpyrimidin-5-yl)methyl)-1H-1,2,3-triazol-4-yl)-N-(2-(phenylsulfonyl)ethyl)ethanaminium 2,2,2-trifluoroacetate,
- 2-(1-((4-amino-2-methylpyrimidin-5-yl)methyl)-1H-1,2,3-triazol-4-yl)-N-(2-(N-(4-fluorobenzyl)sulfamoyl)ethyl)ethanaminium 2,2,2-trifluoroacetate,
- (E)-4-amino-2-methyl-5-((4-(2-((1-(methylamino)-2-nitrovinyl)amino)ethyl)-1H-1,2,3-triazol-1-yl)methyl)pyrimidin-1-ium 2,2,2-trifluoroacetate, and
- 4-amino-5-((4-(((4-(3,5-dicarboxypentanoyl)phenyl)amino)methyl)-1H-1,2,3-triazol-1-yl)methyl)-2-methylpyrimidin-1-ium 2,2,2-trifluoroacetate, respectively.

Examples for esters of the compounds are pyrophosphates. Especially, the pyrophosphates of the compounds according to the formulas (2), (3), (4) and (13) can provide antibacterial activity.

A further aspect of the present invention relates to a compound according to general formula (I) as given as follows and/or racemates, enantiomers, diastereomers, solvates, hydrates, and pharmaceutically acceptable salts and/or esters thereof: 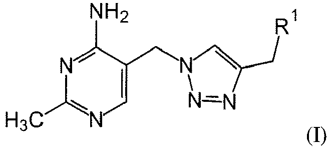 wherein:
- R¹: is selected from the group comprising -OH, -CH₂-NH₂, -(CH₂)₂-OH, -(CH₂)₃-OH, -CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given as follows: 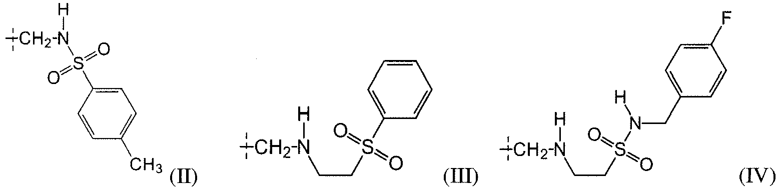 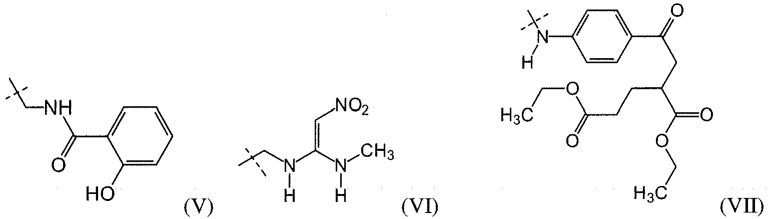 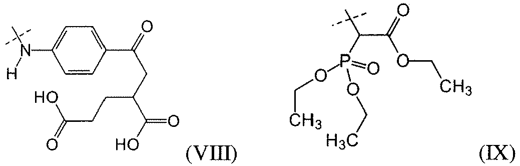
for use as a medicament.

Preferably, the compound for use as a medicament is selected from the triazole thiamines according to the formulas (2) to (17) as given above and/or racemates, enantiomers, diastereomers, solvates, hydrates, and pharmaceutically acceptable salts and/or esters thereof.

In a preferred embodiment, the compound for use as a medicament has the general formula (X) as given as follows and/or racemates, enantiomers, diastereomers, solvates, hydrates, and pharmaceutically acceptable salts and/or esters thereof: 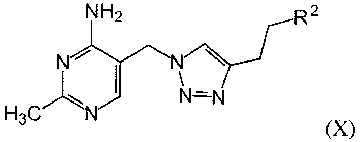 wherein:
- R²: is selected from the group comprising -NH₂, -CH₂-OH, -(CH₂)₂-OH, -O-S(O)₂CH₃, -NH-S(O)₂CH₃, -NH-C(O)-C(O)-CH₃, -NH-C(O)-CH₂-C(O)-O-CH₂-CH₃, and/or sulfones according to formulas (XI), (XII), and (XIII) as given as follows

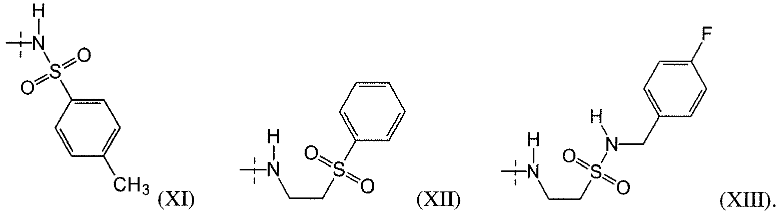

Preferably, the compound for use as a medicament is selected from the triazole thiamines according to formulas (2) to (12) and (13) as given above and/or racemates, enantiomers, diastereomers, solvates, hydrates, and pharmaceutically acceptable salts and/or esters thereof. The compounds according to formulas (2) to (12) and (13) are able to activate the thi-box riboswitch and represent promising antibacterial compounds.

A further aspect of the present invention relates to a compound according to general formula (I) as given as follows and/or racemates, enantiomers, diastereomers, solvates, hydrates, and pharmaceutically acceptable salts and/or esters thereof: 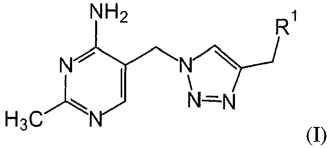 wherein:
- R¹: is selected from the group comprising -CH₂-OH, -OH, -CH₂-NH₂, -(CH₂)₂-OH, -(CH₂)₃-OH, - CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given as follows: 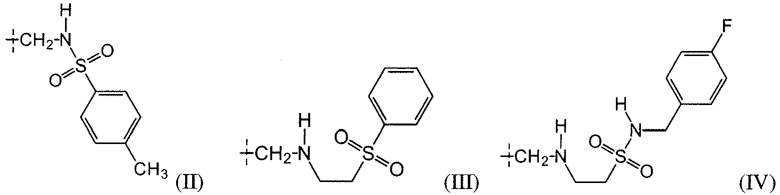 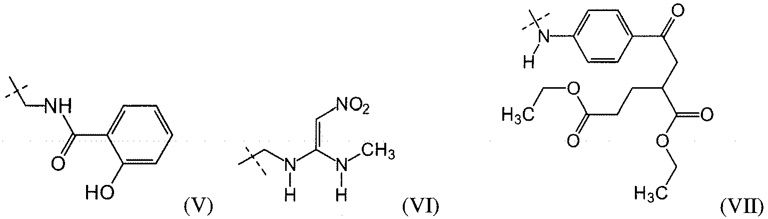 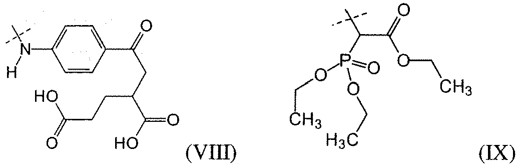
for use in the therapeutic and/or prophylactic treatment of a bacterial infection, particularly for use as an antibiotic.

As used herein, the term "prophylactic treatment" refers to either preventing or inhibiting the development of a clinical condition or disorder or delaying the onset of a pre-clinically evident stage of a clinical condition or disorder. The term "prophylactic treatment" according to the invention is to be understood as meaning that the compositions according to the invention can be applied before symptoms of the bacterial infection are manifest. Especially, the term "prophylactic treatment" is to be understood as meaning a medical treatment. It can be preferred to use the compounds according to the invention in a prophylactic treatment.

In a most preferred embodiment, the compound for use in the therapeutic and/or prophylactic treatment of a bacterial infection, particularly for use as an antibiotic, is the triazole thiamine according to the formula (1) as indicated below and/or racemates, enantiomers, diastereomers, solvates, hydrates, and pharmaceutically acceptable salts and/or esters thereof:

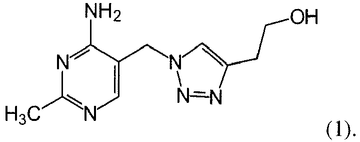

The compound according to the formula (1) is denoted 2-(1-((4-amino-2-methylpyrimidin-5-yl)methyl)-1H-1,2,3-triazol-4-yl)ethanol according to the IUPAC nomenclature.

Further preferred, the compound for use in the therapeutic and/or prophylactic treatment of a bacterial infection, particularly for use as an antibiotic, is selected from the triazole thiamines according to the formulas (2) to (17) as given above and/or racemates, enantiomers, diastereomers, solvates, hydrates, and pharmaceutically acceptable salts and/or esters thereof.

In a preferred embodiment, the compound for use in the therapeutic and/or prophylactic treatment of a bacterial infection, particularly for use as an antibiotic, has the general formula (X) as given as follows and/or racemates, enantiomers, diastereomers, solvates, hydrates, and pharmaceutically acceptable salts and/or esters thereof: 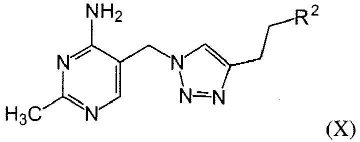 wherein:
- R²: is selected from the group comprising -OH, -NH₂, -CH₂-OH, -(CH₂)₂-OH, -O-S(O)₂CH₃, -NH-S(O)₂CH₃, -NH-C(O)-C(O)-CH₃, -NH-C(O)-CH₂-C(O)-O-CH₂-CH₃, and/or sulfones according to formulas (XI), (XII), and (XIII) as given as follows

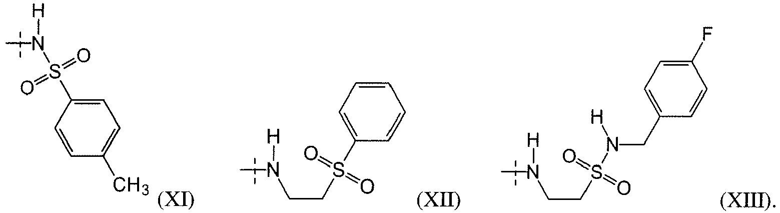

Preferably, the compound is selected from the triazole thiamines according to formulas (1) to (12) and (13) as given above and/or racemates, enantiomers, diastereomers, solvates, hydrates, and pharmaceutically acceptable salts and/or esters thereof, in particular the compound according to the formulas (1). The compounds according to formulas (1) to (12) and (13) especially the compound according to the formulas (1) and (4) represent especially promising antibacterial compounds.

The compounds according to the present invention are usable in the form of solvates, hydrates, and pharmaceutically acceptable salts and/or esters thereof. Preferably, the compounds are usable in the form of pharmaceutically acceptable salts thereof. The term "pharmaceutically acceptable salts" refers to salts prepared from pharmaceutically acceptable non-toxic bases or acids.

When the compound of the present invention is acidic, its corresponding salt of the compounds can be conveniently prepared from pharmaceutically acceptable non-toxic bases, including inorganic bases and organic bases. Preferred salts derived from inorganic bases include ammonium, calcium, magnesium, potassium and sodium salts. Salts derived from pharmaceutically acceptable organic non-toxic bases include salts of primary, secondary, and tertiary amines, as well as cyclic amines.

Preferably, the pharmaceutically acceptable salt is selected from the group of sodium, potassium or ammonium salts. Further preferred are acid addition salts. Preferred acid addition salts usually are hydrochloride salts or hydrochloride hydrates.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic and trifluoroacetic acid and the like. Particularly preferred is trifluoroacetic acid.

Further usable are pharmaceutically acceptable esters of the compounds according to the present invention. The term "pharmaceutically acceptable ester" refers to esters prepared from pharmaceutically acceptable non-toxic ester groups. In an embodiment the esters are physiologically easily hydrolysable esters such as alkyl esters, preferably C1-C4 alkyl esters.

Further examples for esters of the compounds are pyrophosphates. Especially, the pyrophosphates of the compounds according to the formulas (1), (2), (3), (4) and (13) can provide antibacterial activity.

A further aspect of the present invention relates to a pharmaceutical composition comprising as an active ingredient a compound according to general formula (I) as given as follows and/or racemates, enantiomers, diastereomers, solvates, hydrates, and pharmaceutically acceptable salts and/or esters thereof: 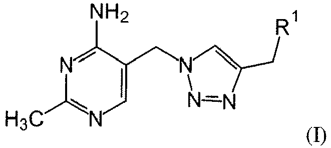 wherein:
R' is selected from the group comprising -OH, -CH₂-NH₂, -(CH₂)₂-OH, -(CH₂)₃-OH, -CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements ccording to formulas (II) to (IX) as given above.

Preferably, the compound is selected from the triazole thiamines according to the formulas (2) to (17) as given above and/or racemates, enantiomers, diastereomers, solvates, hydrates, and pharmaceutically acceptable salts and/or esters thereof.

In a preferred embodiment, the compound has the general formula (X) as given above and/or racemates, enantiomers, diastereomers, solvates, hydrates, and pharmaceutically acceptable salts and/or esters thereof: 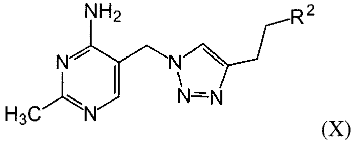 wherein:
- R²: is selected from the group comprising -NH₂, -CH₂-OH, -(CH₂)₂-OH, -O-S(O)₂CH₃, -NH-S(O)₂CH₃, -NH-C(O)-C(O)-CH₃, -NH-C(O)-CH₂-C(O)-O-CH₂-CH₃, and/or sulfones according to formulas (XI), (XII), and (XIII) as given above.

Preferably, the compound is selected from the triazole thiamines according to formulas (2) to (12) and (13) as given above and/or racemates, enantiomers, diastereomers, solvates, hydrates, and pharmaceutically acceptable salts and/or esters thereof.

The compounds and/or racemates, enantiomers, diastereomers, solvates, hydrates, and pharmaceutically acceptable salts and/or esters thereof can be included in pharmaceutical compositions in combination with one or more other therapeutically active compounds. The pharmaceutical composition particularly is usable as an antibiotic.

The pharmaceutical composition especially is usable in the therapeutic and/or prophylactic treatment of a bacterial infection.

A further aspect of the present invention therefore relates to a pharmaceutical composition comprising as an active ingredient a compound according to general formula (I) as given as follows and/or racemates, enantiomers, diastereomers, solvates, hydrates, and pharmaceutically acceptable salts and/or esters thereof: 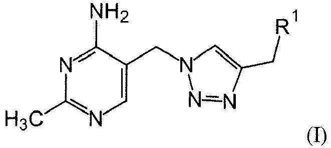 wherein:
- R¹: is selected from the group comprising -CH₂-OH, -OH, -CH₂-NH₂, -(CH₂)₂-OH, -(CH₂)₃-OH, - CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given above
for use in the therapeutic and/or prophylactic treatment of a bacterial infection, particularly for use as an antibiotic.

The pharmaceutical composition for use in the therapeutic and/or prophylactic treatment of a bacterial infection, particularly for use as an antibiotic can comprise as an active ingredient a compound selected from the triazole thiamines according to the formulas (1) to (17) as given above and/or racemates, enantiomers, diastereomers, solvates, hydrates, and pharmaceutically acceptable salts and/or esters thereof.

In a preferred embodiment, the pharmaceutical composition for use in the therapeutic and/or prophylactic treatment of a bacterial infection, particularly for use as an antibiotic can comprise as an active ingredient a compound according to the general formula (X) as follows and/or racemates, enantiomers, diastereomers, solvates, hydrates, and pharmaceutically acceptable salts and/or esters thereof: 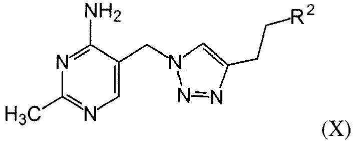 wherein:
- R²: is selected from the group comprising -OH, -NH₂, -CH₂-OH, -(CH₂)₂-OH, -O-S(O)₂CH₃, -NH-S(O)₂CH₃, -NH-C(O)-C(O)-CH₃, -NH-C(O)-CH₂-C(O)-O-CH₂-CH₃, and/or sulfones according to formulas (XI), (XII), and (XIII) as given above.

Preferably, the compound is selected from the triazole thiamines according to formulas (1) to (12) and (13) as given above and/or racemates, enantiomers, diastereomers, solvates, hydrates, and pharmaceutically acceptable salts and/or esters thereof. In a most preferred embodiment, the compound is the triazole thiamine according to the formula (1) which is denoted 2-(1-((4-amino-2-methylpyrimidin-5-yl)methyl)-1H-1,2,3-triazol-4-yl)ethanol according to the IUPAC nomenclature.

A further aspect of the present invention therefore relates to an antibiotic comprising as an active ingredient a compound according to general formula (I) as given as follows and/or racemates, enantiomers, diastereomers, solvates, hydrates, and pharmaceutically acceptable salts and/or esters thereof: 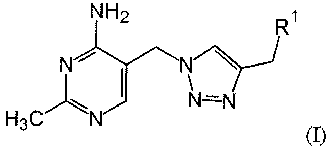 wherein:
- R¹: is selected from the group comprising -CH₂-OH, -OH, -CH₂-NH₂, -(CH₂)₂-OH, -(CH₂)₃-OH, - CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given above.

The antibiotic can comprise as an active ingredient a compound selected from the triazole thiamines according to the formulas (1) to (17) as given above and/or racemates, enantiomers, diastereomers, solvates, hydrates, and pharmaceutically acceptable salts and/or esters thereof.

In a preferred embodiment, the antibiotic can comprise as an active ingredient a compound according to the general formula (X) as follows and/or racemates, enantiomers, diastereomers, solvates, hydrates, and pharmaceutically acceptable salts and/or esters thereof: 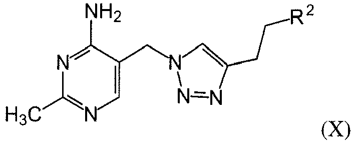 wherein:
- R²: is selected from the group comprising -OH, -NH₂, -CH₂-OH, -(CH₂)₂-OH, -O-S(O)₂CH₃, -NH-S(O)₂CH₃, -NH-C(O)-C(O)-CH₃, -NH-C(O)-CH₂-C(O)-O-CH₂-CH₃, and/or sulfones according to formulas (XI), (XII), and (XIII) as given above.

Preferably, the compound is selected from the triazole thiamines according to formulas (1) to (12) and (13) as given above and/or racemates, enantiomers, diastereomers, solvates, hydrates, and pharmaceutically acceptable salts and/or esters thereof. In a most preferred embodiment, the compound is the triazole thiamine according to the formula (1) which is denoted 2-(1-((4-amino-2-methylpyrimidin-5-yl)methyl)-1H-1,2,3-triazol-4-yl)ethanol according to the IUPAC nomenclature.

Preferably, the pharmaceutical composition comprises a compound according to the invention and/or racemates, enantiomers, diastereomers, solvates, hydrates, and pharmaceutically acceptable salts and/or esters thereof as an active ingredient, a pharmaceutically acceptable carrier and optionally other therapeutic ingredients or adjuvants.

The present invention hence also relates to a pharmaceutical composition wherein the composition comprises: a) as an active ingredient a compound according to general formula (I) as given as follows and/or racemates, enantiomers, diastereomers, solvates, hydrates, and pharmaceutically acceptable salts and/or esters thereof: 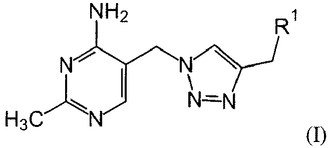 wherein:
- R¹: is selected from the group comprising -OH, -CH₂-NH₂, -(CH₂)₂-OH, -(CH₂)₃-OH, -CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given above
and b) a pharmaceutically acceptable carrier.

The present invention hence also relates to a pharmaceutical composition wherein the composition comprises: a) as an active ingredient a compound according to general formula (I) as given as follows and/or racemates, enantiomers, diastereomers, solvates, hydrates, and pharmaceutically acceptable salts and/or esters thereof: 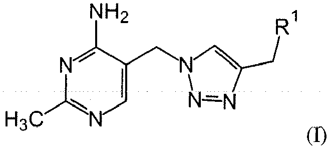 wherein:
- R¹: is selected from the group comprising -CH₂-OH, -OH, -CH₂-NH₂, -(CH₂)₂-OH, -(CH₂)₃-OH, - CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given above
and b) a pharmaceutically acceptable carrier for use in the therapeutic and/or prophylactic treatment of a bacterial infection, particularly for use as an antibiotic.

The pharmaceutical carrier can be, for example, a solid, liquid, or gas. Suitable carriers and adjuvants can be solid or liquid and correspond to the substances ordinarily employed in formulation technology for pharmaceutical formulations. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

The compositions can be suitable for oral, dermal, rectal, topical, and parenteral administration. Parenteral administration includes subcutaneous, intramuscular, and intravenous administration. The pharmaceutical compositions may be conveniently presented in unit dosage form and prepared by any of the methods well known in the art of pharmacy.

The pharmaceutical composition of the present invention can be presented as discrete unit suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion or as a water-in-oil liquid emulsion. Further, the pharmaceutical composition may be administered by controlled release means and/or delivery devices.

For compositions for oral dosage form, convenient pharmaceutical media may be employed. For example, water, glycols, oils, alcohols, flavouring agents, preservatives, colouring agents and the like may be used to form oral liquid preparations such as solutions. Carriers such as starches, sugars, microcrystalline cellulose, diluents, granulating agents, lubricants, binders, disintegrating agents, and the like may be used to form oral solid preparations such as powders, capsules and tablets. Optionally, tablets may be coated by standard aqueous or non aqueous techniques.

Pharmaceutical compositions of the present invention suitable for parenteral administration may be prepared as solutions or suspensions of the active compounds in water. A suitable excipient can be included such as, for example, hydroxypropylcellulose. Dispersions can also be prepared in glycerol, liquid polyethylene glycols, and mixtures thereof in oils. Pharmaceutical compositions of the present invention suitable for injectable use include sterile aqueous solutions or dispersions. Further, a preservative can be included to prevent the growth of microorganisms.

The pharmaceutical compositions of the present invention can be in a form suitable for topical use such as, for example, an aerosol, cream, ointment, lotion, dusting powder, or the like. Further, the compositions can be in a form suitable for use in transdermal devices. The pharmaceutical composition may also be prepared in powder or liquid concentrate form. The pharmaceutical composition of the present invention can include one or more additional carrier ingredients such as diluents, buffers, flavouring agents, binders, surface-active agents, thickeners, lubricants, preservatives and the like.

The pharmaceutical composition may be produced under sterile conditions using standard pharmaceutical techniques well known to those skilled in the art.

The present invention also relates to the use of a compound according to general formula (I) as given as follows and/or racemates, enantiomers, diastereomers, solvates, hydrates, and pharmaceutically acceptable salts and/or esters thereof: 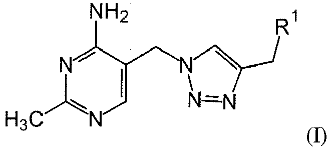 wherein:
- R¹: is selected from the group comprising -OH, -CH₂-NH₂, -(CH₂)₂-OH, -(CH₂)₃-OH, -CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given above
for the manufacture of a medicament.

It is a particular advantage of the compounds according to the invention is that the compounds are especially usable for the manufacture of an antibiotic, particularly against Haemophilus influenza and other bacteria, which regulate essential genes of TPP-biosynthesis via thi-box riboswitches.

Preferably, triazole thiamines according to the formulas (2) to (17) as given above and/or racemates, enantiomers, diastereomers, solvates, hydrates, and pharmaceutically acceptable salts and/or esters thereof are usable for the manufacture of a medicament.

In a preferred embodiment, the compound usable for the manufacture of a medicament has the general formula (X) as given as follows and/or racemates, enantiomers, diastereomers, solvates, hydrates, and pharmaceutically acceptable salts and/or esters thereof: 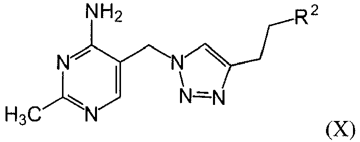 wherein:
- R²: is selected from the group comprising -NH₂, -CH₂-OH, -(CH₂)₂-OH, -O-S(O)₂CH₃, -NH-S(O)₂CH₃, -NH-C(O)-C(O)-CH₃, -NH-C(O)-CH₂-C(O)-O-CH₂-CH₃, and/or sulfones according to formulas (XI), (XII), and (XIII) as given above.

Preferably, the compound usable for the manufacture of a medicament is selected from the triazole thiamines according to formulas (2) to (12) and (13) as given above and/or racemates, enantiomers, diastereomers, solvates, hydrates, and pharmaceutically acceptable salts and/or esters thereof.

The present invention also relates to the use of a compound according to general formula (I) as given as follows and/or racemates, enantiomers, diastereomers, solvates, hydrates, and pharmaceutically acceptable salts and/or esters thereof: 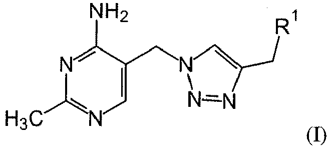 wherein:
- R¹: is selected from the group comprising -CH₂-OH, -OH, -CH₂-NH₂, -(CH₂)₂-OH, -(CH₂)₃-OH, - CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given above
for the manufacture of a medicament for the therapeutic and/or prophylactic treatment of a bacterial infection, particularly for the manufacture of an antibiotic.

Preferably, triazole thiamines according to the formulas (1) to (17) as given above and/or racemates, enantiomers, diastereomers, solvates, hydrates, and pharmaceutically acceptable salts and/or esters thereof are usable for the manufacture of a medicament for the therapeutic and/or prophylactic treatment of a bacterial infection, particularly for the manufacture of an antibiotic.

In a preferred embodiment, a compound according to the general formula (X) as follows and/or racemates, enantiomers, diastereomers, solvates, hydrates, and pharmaceutically acceptable salts and/or esters thereof: 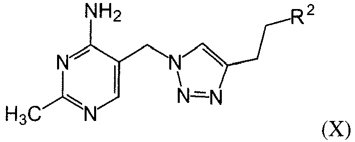 wherein:
- R²: is selected from the group comprising -OH, -NH₂, -CH₂-OH, -(CH₂)₂-OH, -O-S(O)₂CH₃, -NH-S(O)₂CH₃, -NH-C(O)-C(O)-CH₃, -NH-C(O)-CH₂-C(O)-O-CH₂-CH₃, and/or sulfones according to formulas (XI), (XII), and (XIII) as given above
is usable for the manufacture of a medicament for the therapeutic and/or prophylactic treatment of a bacterial infection, particularly for the manufacture of an antibiotic.

Preferably, triazole thiamines according to formulas (1) to (12) and (13) as given above and/or racemates, enantiomers, diastereomers, solvates, hydrates, and pharmaceutically acceptable salts and/or esters thereof are usable for the manufacture of a medicament for the therapeutic and/or prophylactic treatment of a bacterial infection, particularly for the manufacture of an antibiotic. In a most preferred embodiment the triazole thiamine according to the formula (1) which is denoted 2-(1-((4-amino-2-methylpyrimidin-5-yl)methyl)-1H-1,2,3-triazol-4-yl)ethanol according to the IUPAC nomenclature is usable for the manufacture of a medicament for the therapeutic and/or prophylactic treatment of a bacterial infection, particularly for the manufacture of an antibiotic.

The compounds of the present invention may be prepared using a variety of processes well known by a person skilled in the art.

A further aspect of the present invention relates to a method for preparing a compound according to any one of the foregoing claims comprising the steps of:
a) Providing an azide functionalised pyrimidine according to formula (XIV) 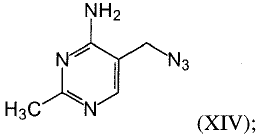
b1) Reacting the azide functionalised pyrimidine according to formula (XIV) with an alkyne according to formula (XV) 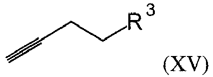 wherein R³ is selected from the group comprising -OH, -NH₂, -CH₂-OH, -(CH₂)₂-OH, and -O-S(O)₂CH₃ to a compound according to formula (XVI) 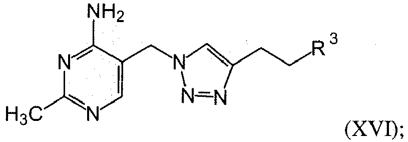 and
c1) Reacting the compound according to formula (XVI) wherein R³ is -NH₂ of step b1) with a compound C1-SO₂-R⁴ wherein R⁴ is selected from the group comprising -CH₃ or -C₆H₄-CH₃ to a compound according to formula (XVII) 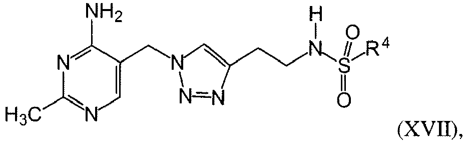 or
c2) Reacting the compound according to formula (XVI) wherein R³ is -NH₂ of step b1) with a compound HOOC-⁵ wherein R⁵ is selected from the group comprising -CHO, -CH₂-COOC₂H₅ or -C₆H₄-OH to a compound according to formula (XVIII) 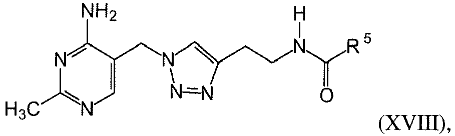 or
c3) Reacting the compound according to formula (XVI) wherein R³ is -NH₂ of step b1) with a compound H₂C=CH-R⁶ to a compound according to formula (XIX) 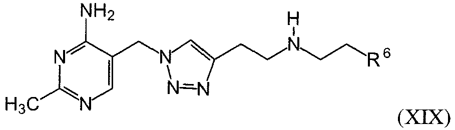 wherein R⁶ is selected from the group comprising structural elements according to formulas (XX), and (XXI) as given as follows 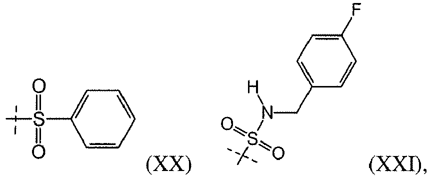 or
c4) Reacting the compound according to formula (XVI) wherein R³ is -NH₂ of step b1) with the compound C(S-CH₃)(NH-CH₃)CH-NO₂ to the compound according to formula (14) 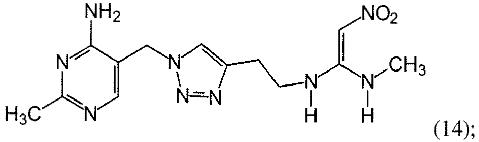 or
b2) Reacting the azide functionalised pyrimidine according to formula (XIV) with an alkyne according to formula (XXII) 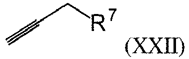 to a compound according to formula (XXIII) 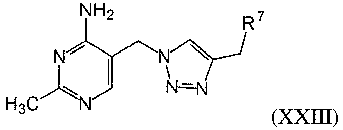 wherein R⁷ is -OH or selected from the group comprising structural elements according to formulas (VII) or (IX) as given as follows

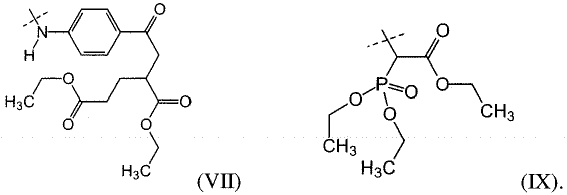

The azide functionalised pyrimidine according to formula (XIV) may be prepared using processes well known by a person skilled in the art.

The reaction of the compound according to formula (XVI) wherein R³ is -NH₂ with a compound HOOC-R⁵ to a compound according to formula (XVIII) of step c2) preferably is carried out as a two-step reaction in the presence of an equivalent amount of triethylamine in dimethylformamide. The hydrochloride salt of the amine is converted to the free base through use of one equivalent of triethylamine and reacted with the carboxylic acid in the presence of a coupling agent. The product can be purified using high-performance liquid chromatography using dimethylformamide.

The reaction of the compound according to formula wherein R³ is -NH₂ with the compound C(S-CH₃)(NH-CH₃)CH-NO_{Z} to the compound according to formula (14) of step c4) preferably is carried out in the presence of an equivalent amount of triethylamine in dimethylformamide. The product can be purified using high-performance liquid chromatography using dimethylformamide.

The reaction of the azide functionalised pyrimidine according to formula (XIV) with an alkyne according to formula (XXII) to a compound according to formula (XXIII) in step b2) preferably is carried using copper sulphate and sodium ascorbate. A preferred solvent is a mixture of t-butyl alcohol and water, preferably a 3:1 mixture of t-butyl alcohol and water. The product can be purified using high-performance liquid chromatography.

The compound according to formula (16) is obtainable by refluxing the compound according to formula (15) in dilute HCl. The product can be purified using high-performance liquid chromatography.

A further aspect of the present invention relates to a method of treating a bacterial infection, the method comprising administering to the subject a therapeutically effective amount of a compound according to the general formula (I) as given as follows and/or racemates, enantiomers, diastereomers, solvates, hydrates, and pharmaceutically acceptable salts and/or esters thereof: 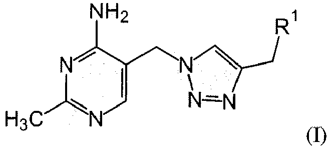 wherein:
- R¹: is selected from the group comprising -CH₂-OH, -OH, -CH₂-NH₂, -(CH₂)₂-OH, -(CH₂)₃-OH, - CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given above.

The term "therapeutically effective amount" is used herein to mean an amount or dose sufficient to cause an improvement in a clinically significant condition in the subject.

Unless otherwise defined, the technical and scientific terms used herein have the same meaning as commonly understood by one of ordinary skill in the art to which this invention belongs.

The Examples which follow serve to illustrate the invention in more detail but do not constitute a limitation thereof.

The figures show:
- Figure 1: the Expression of β-galactosidase in the presence of thiamine (T) as a positive control, the compounds according to formulas (1), (4), (13), (2), and (3), respectively, pyrithiamine (PT), and an untreated control (0) in *E. coli* DH5aZ' cells.
- Figure 2: the Expression of β-galactosidase in the presence of thiamine (T) as a positive control, the compounds according to formulas (1), (4), (13), (2), and (3), respectively, and pyrithiamine (PT), and an untreated control (0) in *E. coli* MG1655 cells.
- Figure 3: the effect on β-galactosidase expression exhibited by thiamine (T) as a positive control and the compounds according to formulas (5), (6), the trifluoroacetate of the compound according to formula (7), compound (8), compound (9), the trifluoroacetates of the compounds according to formulas (10) and (11), respectively, and compound (12) and an untreated control (0) in *E*. *coli* DH5aZ' cells.

Unless stated otherwise, a purification of the compounds was carried out either using high performance liquid chromatography (HPLC) or using flash column chromatography, a variant of column chromatography. Flash column chromatography was carried out using Merck silica gel 60 (40 - 63 µm). Pressure was achieved by compressed air. The mobile phase, column diameter (Ø), filling level of silica gel, and volume of fractions was adjusted to experimental conditions.

The solvents were distilled before use. Chemicals were purchased from Acros and Sigma-Aldrich and used without further purification, unless stated otherwise.

### Example 1

### Preparation of the chloride salt of the compound according to formula (4) denoted 2-{1-[(4-amino-2-methyl-5-pyrimidinyl)methyl]-1H-1,2,3-triazol-4-yl}ethanaminium chloride

### step 1.1 Preparation of 5-Azidomethyl-2-methylpyrimidin-4-amine

To a solution of thiamine chloride (10.3 g, 30.6 mmol) and sodium azide (4.9 g, 75.4 mmol) in water (100 mL) was added sodium sulfite (0.38 g, 3.0 mmol) and the mixture was stirred at 65°C for 5 h. Citric acid (21.0g, 100 mmol, to pH ≈ 4) was added and the aqueous solution was washed with dichloromethane. Potassium carbonate (pH ≈ 8) was added and this was extracted with ethyl acetate and the combined organic layers were washed with brine, dried over MgSO₄ and evaporated under reduced pressure. The solid residue recrystallised from ethyl acetate/hexane gave the azide as fine needles (3.15g, 63 % yield).

### step 1.2 Preparation of 3-butynyl methanesulfonate

3-Butyn-1-ol (11.72 g, 0.17 mol) was dissolved in dichloromethane (DCM) (80 mL) and cooled to 0° C, and Et₃N (35.3 mL, 0.25 mol) and 4-dimethylaminopyridne (DMAP, 800 mg) was added in one portion, followed by addition of methanesulfonyl chloride (19.5 mL, 0.25 mol). The reaction mixture was stirred for 2 h at 0 °C and for another 3 h at 25 °C. The reaction was cooled again to 0 °C, quenched with addition of 50 mL water, and extracted 3 times with 100 mL Et₂O. The combined organic phase was washed with aqueous NH₄Cl, aqueous NaHCO₃, brine, and dried over Na₂SO₄ overnight. After removal of volatile solvents by rotary evaporation, the residue was subjected to silica gel flash column chromatography to furnish the desired mesylate (14.8 g, 62 % yield).

### step 1.3 Preparation of 3-butyn-1-aminium chloride

5.5 g 3-Butynyl methanesulfonate of step 1.2 (24.5 mmol) was dissolved in 100 mL anhydrous dimethylformamide (DMF), and sodium azide (4 g, 61.4 mmol) added in portions. The reaction mixture was heated to 70 °C and stirred for 3.5 h, cooled to 25 °C and extracted with Et₂O (50 mL × 3). The combined organic phase was washed with water, brine, and dried over Na₂SO₄. Volatile solvents were removed by rotary evaporation. The resulting residue containing 3-butyn-1-azide was used without further purification and was mixed with triphenyl phosphine (6.44 g, 24.4 mmol) in Et₂O (100 mL) at 0° C and stirred for 2 h. Water (3.9 mL) was added and the resulting mixture stirred for an additional 20 h at 25 °C. The mixture was poured into 1N HCl (25 mL) and the aqueous layer extracted with Et₂O (10 mL × 3). The remaining aqueous layer was concentrated under reduced pressure rotary evaporation to give 1.25 g 3-butyn-1-aminium chloride (49 % yield for two steps).

### step 1.4 Preparation of 2-{1-[(4-amino-2-methyl-5-pyrimidinyl)methyl]-1H-1,2,3-triazol-4-yl}ethanaminium chloride

To a stirred solution of 3-butyn-1-aminium chloride of step 1.3 (400 mg, 3.79 mmol) and 5-Azidomethyl-2-methylpyrimidin-4-ylamine of step 1.1 (500 mg, 3.04 mmol) in tert-butanol/water (5 mL; 2: 1) were added sodium ascorbate (60 mg, 0.30 mmol) and CuSO₄·5H₂O (8 mg, 0.32 mmol). The reaction mixture was stirred at room temperature (20±2°C) for 16 h. This was evaporated under reduced pressure to give the crude material as a brown residue. This was purified by flash column chromatography (MeOH:EtOAc; 10:90, R_{f} 0.09) to give 2-{1-[(4-amino-2-methyl-5-pyrimidinyl)methyl]-1H-1,2,3-triazol-4-yl}ethanaminium chloride as a brown solid (512 mg, 60 % yield).

### Example 2

### Preparation of the trifluoroacetate of the compound according to formula (7),d 4-amino-2-methyl-5-[(4-{2-[(methylsulfonyl)amino]ethyl}-1H-1,2,3-triazol-1-yl)methyl]pyrimidin-1-ium trifluoroacetate

2-{1-[(4-Amino-2-methyl-5-pyrimidinyl)methyl]-1H-1,2,3-triazol-4-yl}ethanaminium chloride as prepared in example 1 (100 mg, 0.43 mmol) was dissolved in DMF (2 mL, dry) to which was added triethylamine (44 mg, 0.43 mmol). Methane sulfonyl chloride (49 mg, 0.43 mmol) was added dropwise and left to stir for 16 h. The resulting solution was subjected to HPLC purification at a flow rate of 6 mL/min and a retention time of 8 min to obtain the desired product (25 mg, 14 % yield).

### HPLC procedure:

| Time | % Water (with 0.1% trifluoroacetic acid (TFA)) | % acetonitrile (MeCN) (with 0.1% TFA) |
|---|---|---|
| 0 | 100 | 0 |
| 20 | 70 | 30 |

### Example 3

### Preparation of the trifluoroacetate of the compound according to formula (6), 4-amino-2-methyl-5-{[4-(2-{[(4-methylphenyl) sulfonyl]amino}ethyl)-1H-1,2,3-triazol-1-yl]methyl}pyrimidine-1-ium trifluoroacetate

2-{1-[(4-Amino-2-methyl-5-pyrimidinyl)methyl]-1H-1,2,3-triazol-4-yl}ethanaminium chloride as prepared in example 1 (100 mg, 0.43 mmol) was dissolved in DMF (2 mL, dry) to which was added triethylamine (44 mg, 0.43 mmol). p-Toluene sulfonyl chloride (82 mg, 0.43 mmol) was added in small portions and left to stir for 16 h. The resulting solution was subjected to HPLC purification at a flow rate of 6 mL/min and a retention time of 11 min to obtain the desired product (82 mg, 38 % yield).

### HPLC procedure:

| Time | % Water (with 0.1% TFA) | % MeCN (with 0.1% TFA) |
|---|---|---|
| 0 | 100 | 0 |
| 8 | 70 | 30 |
| 20 | 70 | 30 |

### Example 4

### Preparation of the compound according to formula (9) 3-[(2-{1-[(4-amino-2-methyl-5-pyrimidinyl)methyl]-1H-1,2,3-triazol-4-yl}ethyl)amino]-3-oxopropanoate

2-{1-[(4-Amino-2-methyl-5-pyrimidinyl)methyl]-1H-1,2,3-triazol-4-yl}ethanaminium chloride as prepared in example 1 (100 mg, 0.428 mmol) was dissolved in DMF (2 mL, dry) to which was added triethylamine (44 mg, 0.430 mmol). Mono-ethyl malonate (113.52 mg, 0.86 mmol) was dissolved in DMF (2 mL, dry) and cooled to 0°C and to this was added N,N'-Dicyclohexylcarbodiimide (DCC) (178 mg, 0.86 mmol). The cooled pyruvic acid mixture was slowly added to the cooled amine mixture and left to stir for 4 h. The solid was filtered off and the resulting solution was subjected to HPLC purification at a flow rate of 6 mL/min and a retention time of 6 min to obtain the desired product (24 mg, 16 % yield).

### HPLC procedure:

| Time | % Water (with 0.1% Formic Acid) | % MeCN (with 0.1% Formic Acid) |
|---|---|---|
| 0 | 95 | 5 |
| 10 | 80 | 20 |
| 15 | 70 | 30 |
| 20 | 70 | 30 |

### Example 5

### Preparation of the trifluoroacetate of the compound according to formula (12), 4-amino-5-[(4-{2-[(2-hydroxybenzoyl)amino]ethyl}-1H-1,2,3-triazol-1-yl)methyl]-2-methylpyrimidin-1-ium trifluoroacetate

2-{1-[(4-Amino-2-methyl-5-pyrimidinyl)methyl]-1H-1,2,3-triazol-4-yl}ethanaminium chloride as prepared in example 1 (100 mg, 0.43 mmol) was dissolved in DMF (2 mL, dry) to which was added triethylamine (44 mg, 0.43 mmol). In a separate flask, the salicylic acid (70 mg, 0.51 mmol) was dissolved in DMF (1 mL, dry) and to this was added N,N'-Dicyclohexylcarbodiimide (DCC) (90 mg, 0.43 mmol) and triethylamine (44 mg, 0.43 mmol). The amine solution was then slowly added to the carboxylic acid solution at 0 °C and left stirring at room temperature for 16 h. The resulting solution was subjected to HPLC purification at a flow rate of 6 mL/min and a retention time of 10.1 min to obtain the desired product (100 mg, 51 % yield).

### HPLC procedure:

| Time | % Water (with 0.1% TFA) | % MeCN (with 0.1% TFA) |
|---|---|---|
| 0 | 85 | 15 |
| 5 | 85 | 15 |
| 10 | 70 | 30 |
| 20 | 70 | 30 |

### Example 6

### Preparation of the compound according to formula (7) N-(2-{1-[(4-amino-2-methyl-5-pyrimidinyl)methyl]-1H-1,2,3-triazol-4-yl}ethyl)-2-oxopropanamide

2-{1-[(4-Amino-2-methyl-5-pyrimidinyl)methyl]-1H-1,2,3-triazol-4-yl}ethanaminium chloride as prepared in example 1 (50 mg, 0.214 mmol) was dissolved in DMF (1 mL, dry) to which was added triethylamine (22 mg, 0.214 mmol). Pyruvic acid (38 mg, 0.43 mmol) was dissolved in DMF (1 mL, dry) and cooled to 0°C and to this was added DCC (88.6 mg, 0.43 mmol). The cooled pyruvic acid mixture was slowly added to the cooled amine mixture and left to stir for 4 h. The solid was filtered off and the resulting solution was subjected to HPLC purification at a flow rate of 6 mL/min and a retention time of 3 min to obtain the desired product as an oil (21 mg, 33 % yield).

### HPLC procedure:

| Time | % Water (with 0.1% Formic Acid) | % MeCN (with 0.1% Formic Acid) |
|---|---|---|
| 0 | 95 | 5 |
| 10 | 80 | 20 |
| 15 | 70 | 30 |
| 20 | 70 | 30 |

### Example 7

### Preparation of the bis(trifluoroacetate) of the compound according to formula (10), 4-amino-2-methyl-5-{[4-(2-{[2-(phenylsulfonyl)ethyl]ammonio}ethyl)-1H-1,2,3-triazol-1-yl]methyl}pyrimidin-1-ium bis(trifluoroacetate)

2-{1-[(4-Amino-2-methyl-5-pyrimidinyl)methyl]-1H-1,2,3-triazol-4-yl}ethanaminium chloride as prepared in example 1 (50 mg, 0.214 mmol) was dissolved in DMF (1 mL, dry) to which was added triethylamine (22 mg, 0.214 mmol) and after 30 min of stirring phenyl vinyl sulfone (36 mg, 0.214 mmol) was added. The reaction mixture was left for 16 h. The reaction mixture was subjected to HPLC purification at a flow rate of 6 mL/min and a retention time of 7.1 min to obtain the desired product (36 mg, 27 % yield).

### HPLC procedure:

| Time | % Water (with 0.1% TFA) | % MeCN (with 0.1% TFA) |
|---|---|---|
| 0 | 95 | 5 |
| 10 | 80 | 20 |
| 15 | 70 | 30 |
| 20 | 70 | 30 |

### Example 8

### Preparation of the bis(trifluoroacetate) of the compound according to formula (11), 4-amino-5-[(4-{2-[(2-{[(4-fluorobenzyl)amino]sulfonyl}ethyl)ammonio]ethyl}-1H-1,2,3-triazol-1-yl)methyl]-2-methylpyrimidin-1-ium bis(trifluoroacetate)

### step 8.1

### Preparation of N-(4-Fluorobenzyl)ethylenesulfonamide:

4-Fluorobenzylamine (1.00 g, 7.99 mmol) was dissolved in DCM (5 mL, dry) at 0°C with stirring under N₂ to which N-methyl morpholine (808 mg; 879 µL; 7.99 mmol) was added with stirring: A solution of 2-chloroethanesulfonyl chloride (1.30 g, 840 µL, 7.99 mmol) dissolved in DCM (5 mL, dry) was added at 0°C with stirring under N₂. After which time the reaction mixture was left stirring at room temperature overnight. The reaction mixture was extracted with dilute hydrochloric acid and the organic layers were collected, dried (MgSO₄), filtered and the solvent removed under reduced pressure. The crude product was purified by column chromatography (ethyl acetate/n-hexane 1/3, R_{F}=0.2). The product was obtained as a white solid (0.927 g, 54%).

### step 8.2

2-{1-[(4-Amino-2-methyl-5-pyrimidinyl)methyl]-1H-1,2,3-triazol-4-yl}ethanaminium chloride as prepared in example 1 (50 mg, 0.214 mmol) was dissolved in DMF (1 mL, dry) to which was added triethylamine (22 mg, 0.214 mmol) and after 30 min of stirring N-(4-fluorobenzyl)ethylene-sulfonamide of step 8.1 (40 mg, 0.214 mmol) was added. The reaction mixture was heated to 50°C and left for 16 h. The reaction mixture was subjected to HPLC purification at a flow rate of 6 mL/min and a retention time of 12.5 min to obtain the desired product (30 mg, 26 % yield).

### HPLC procedure:

| Time | % Water (with 0.1% TFA) | % MeCN (with 0.1% TFA) |
|---|---|---|
| 0 | 95 | 5 |
| 10 | 80 | 20 |
| 15 | 70 | 30 |
| 20 | 70 | 30 |

### Example 9

### Preparation of the compound according to formula (5) 2-{ 1-[(4-amino-2-methyl-5-pyrimidinyl)methyl]-1H-1,2,3-triazol-4-yl }ethyl methanesulfonate

To a stirred solution of 3-butynyl methanesulfonate as prepared according to step 1.2 of example 1 (700 mg, 3.11 mmol) and 5-Azidomethyl-2-methylpyrimidin-4-ylamine as prepared according to step 1.1 of example 1 (500 mg, 3.04 mmol) in tert-butanol/ water (5 mL; 2 : 1) were added sodium ascorbate (60 mg, 0.30 mmol) and CuSO₄·5H₂O (8 mg, 0.32 mmol). The reaction mixture was stirred at room temperature for 16 h. The crude mixture was evaporated under reduced pressure and the solid residue was purified by flash column chromatography (MeOH:EtOAc; 10:90, R_{f} 0.15). The desired material was obtained as a white powder (500 mg, 53 % yield).

### Example 10

### Preparation of the compound according to formula (1) 2-[1-(4-Amino-2-methylpyrimidin-5-ylmethyl)-1H-[1,2,3]triazol-4-yl]ethanol

To a stirred solution of 3-butynol (907 µl, 12.0 mmol) and 5-Azidomethyl-2-methylpyrimidin-4-ylamine as prepared according to step 1.1 of example 1 (1.968 g, 12.0 mmol) in tert-butanol/ water (12 mL; 2 : 1) were added sodium ascorbate (238 mg, 1.2 mmol) and CuSO₄·5H₂O (30 mg, 0.12 mmol). The reaction mixture was stirred at room temperature for 16 h. The crude mixture was evaporated under reduced pressure and the solid residue was dissolved in 1-butanol. The organic layer was washed with aqueous potassium carbonate (0.1 M) and then with brine, dried over MgSO₄ and evaporated under reduced pressure to give a white solid. Recrystallisation from 2-propanol/hexane gave the triazole as fine needles (2.27 g, 81 % yield).

### Example 11

### Preparation of the compound according to formula (2) 3-{ 1-[(4-amino-2-methyl-5-pyrimidinyl)methyl]-1H-1,2,3-triazol-4-yl }-1-propanol

To a stirred solution of 4-pentynol (150 µl, 1.61 mmol) and 5-Azidomethyl-2-methylpyrimidin-4-ylamine as prepared according to step 1.1 of example 1 (250 mg, 1.52 mmol) in tert-butanol/ water (5 mL; 2 : 1) were added sodium ascorbate (30 mg, 0.15 mmol) and CuSO₄·5H₂O (4 mg, 0.016 mmol). The reaction mixture was stirred at room temperature for 16 h. The crude mixture was evaporated under reduced pressure and the solid residue was purified by flash column chromatography (MeOH:EtOAc; 10:90, R_{f} 0.17). The desired material was obtained as a white powder (310 mg, 71 % yield).

### Example 12

### Preparation of the compound according to formula (3) 4-{ 1-[(4-amino-2-methyl-5-pyrimidinyl)methyl]-1 H-1,2,3-triazol-4-yl }-1-butanol

To a stirred solution of 5-hexynol (300 µl, 3.0 mmol) and 5-Azidomethyl-2-methylpyrimidin-4-ylamine as prepared according to step 1.1 of example 1 (500 mg, 3.04 mmol) in tert-butanol/ water (5 mL; 2 : 1) were added sodium ascorbate (60 mg, 0.30 mmol) and CuSO₄.5H₂O (8 mg, 0.32 mmol). The reaction mixture was stirred at room temperature for 16 h. The crude mixture was evaporated under reduced pressure and the solid residue was purified by flash column chromatography (MeOH:EtOAc; 10:90, R_{f} 0.19). The desired material was obtained as a white powder (644 mg, 82 % yield).

### Example 13

### Preparation of the compound according to formula (13) 1-[(4-amino-2-methyl-5-pyrimidinyl)methyl]-1H-1,2,3-triazol-4-yl} methanol

To a stirred solution of 2-propynol (90.3 mg, 1.61 mmol) and 5-Azidomethyl-2-methylpyrimidin-4-ylamine as prepared according to step 1.1 of example 1 (250 mg, 1.52 mmol) in tert-butanol/ water (5 mL; 2 : 1) were added sodium ascorbate (30 mg, 0.15 mmol) and CuSO₄·5H₂O (4 mg, 0.016 mmol). The reaction mixture was stirred at room temperature for 16 h. The crude mixture was evaporated under reduced pressure and the solid residue was purified by flash column chromatography (MeOH:EtOAc; 10:90, R_{f} 0.16). The desired material was obtained as a light brown powder 204 mg, 61 % yield).

### Example 14

### Preparation of the trifluoroacetate of the compound according to formula (14), 4-amino-2-methyl-5-{[4-(2-{[(E)-1-(methylamino)-2-nitroethenyl]amino}ethyl)-1H-1,2,3-triazol-1-yl]methyl}pyrimidin-1-ium trifluoroacetate

2-{ 1-[(4-amino-2-methyl-5-pyrimidinyl)methyl]-1H-1,2,3-triazol-4-yl}ethanaminium chloride as prepared in example 1 (50 mg, 0.214 mmol) was dissolved in DMF (1 mL, dry) to which was added triethylamine (22 mg, 0.214 mmol) and after 30 min of stirring (E)-N-methyl-1-(methylthio)-2-nitroethenamine (32 mg, 0.214 mmol) was added. The reaction mixture was left for 16 h. The reaction mixture was subjected to HPLC purification at a flow rate of 6 mL/min and a retention time of 8.2 min to obtain the desired product (12 mg, 13 % yield).

### HPLC procedure:

| Time | % Water (with 0.1% TFA) | % MeCN (with 0.1% TFA) |
|---|---|---|
| 0 | 95 | 5 |
| 10 | 80 | 20 |
| 15 | 70 | 30 |
| 20 | 70 | 30 |

### Example 15

### Preparation of the compound according to formula (15) diethyl 2-(2-{4-[({1-[(4-amino-2-methyl-5-pyrimidinyl)methyl]-1H-1,2,3-triazol-4-yl}methyl)amino]phenyl}-2-oxoethyl)pentanedioate

To a stirred solution of diethyl 2-{2-oxo-2-[4-(2-propynylamino)phenyl]ethyl}pentanedioate (579 mg, 1.61 mmol) and 5-Azidomethyl-2-methylpyrimidin-4-ylamine as prepared according to step 1.1 of example 1 (250 mg, 1.52 mmol) in tert-butanol/ water (5 mL; 2 : 1) were added sodium ascorbate (30 mg, 0.15 mmol) and CuSO₄·5H₂O (4 mg, 0.016 mmol). The reaction mixture was stirred at room temperature for 16 h. The crude mixture was evaporated under reduced pressure and the solid residue was purified by flash column chromatography (MeOH:EtOAc; 10:90, R_{f} 0.21). The desired material was obtained as a light brown powder (263 mg, 33 % yield).

### Example 16

### Preparation of the compound according to formula (16) 4-amino-5-[(4-([4-(3,5-dicarboxy-pentanoyl)anilino]methyl}-1H-1,2,3-triazol-1-yl)methyl]-2-methylpyrimidin-1-ium trifluoroacetate

Diethyl 2-(2-{4-[({1-[(4-amino-2-methyl-5-pyrimidinyl)methyl]-1H-1,2,3-triazol-4-yl}methyl)amino]phenyl}-2-oxoethyl)pentanedioate as prepared according example 15 (100 mg, 0.19 mmol) was dissolved in dilute HCl (1 mL) and left to reflux for 4 hrs. The reaction mixture was purified directly by HPLC at a flow rate of 6 mL/min and a retention time of 8.22 min. The desired material was obtained as a white powder (50 mg, 45 % yield).

### HPLC procedure:

| Time | % Water (with 0.1% TFA) | % MeCN (with 0.1% TFA) |
|---|---|---|
| 0 | 95 | 5 |
| 24 | 50 | 50 |
| 30 | 30 | 70 |

### Example 17

### Preparation of the compound according to formula (17) 4-amino-5-({4-[2-(diethoxyphosphoryl)-3-ethoxy-3-oxopropyl]-1H-1,2,3-triazol-1-yl}methyl)-2-methylpyrimidin-1-ium trifluoroacetate

To a stirred solution of ethyl 2-(diethoxyphosphoryl)-4-pentynoate (80 mg, 0.304 mmol) and 5-Azidomethyl-2-methylpyrimidin-4-ylamine as prepared according to step 1.1 of example 1 (50 mg, 0.304 mmol) in tert-butanol/ water (5 mL; 2 : 1) were added sodium ascorbate (6 mg, 0.03 mmol) and CuSO₄·5H₂O (0.8 mg, 0.0032 mmol). The reaction mixture was stirred at room temperature for 4 h. The solvent was removed by rotary evaporation and the resulting residue was dissolved in DMF (1 mL, dry) and purified by HPLC at a flow rate of 6 mL/min and a retention time of 31.2 min. The desired material was obtained as an off white solid (30 mg, 23 % yield).

### HPLC procedure:

| Time | % Water (with 0.1% TFA) | % MeCN (with 0.1% TFA) |
|---|---|---|
| 0 | 97 | 3 |
| 25 | 50 | 50 |
| 30 | 30 | 70 |
| 35 | 97 | 3 |

### Example 18

Investigation of in vivo effects of the compounds according to formulas (1), (2), (3), (4), and (13) by the β-galactosidase reportergene assay in E. coli DH5aZ' cells

A reportergene assay was used to investigate the effect of the compounds on gene expression of β-galactosidase. In this assay, bacteria were grown which contain a plasmid that encodes the thiM riboswitch, the thi-box riboswitch found in E.coli, cloned infront of the β-galactosidase gene. This riboswitch causes the inhibition of translation initiation upon thiaminepyrophosphate (TPP) binding, leading to a decrease in β-galactosidase expression level. The β-galactosidase expression level is monitored by cleavage of an artificial substrate, O-nitrophenyl-β-galactopyranoside (ONPG), leading to the generation of a colored product that is measured photometrically at 420 nm.

The β-galactosidase reporter gene assay was performed in E. coli DH5αZ' [pRS414.2thiM] cells obtained from Hermann Bujard (next to DH5α inherent genomic alterations (12: F-, Δ(argF-lac) 169, ϕ80dlacZ58(M15), ΔphoA8, glnV44(AS), λ, deoR481, rfbc1?, yrA96(NalR), recA1, endA1, thi-1, hsdR17, containing mutations integrated according to: Lutz et al. NAR 1997, Vol. 25, No. 6; Diederich,L., Rasmussen,L.J. and Messer,W. (1992) Plasmid 28, 14-24.). These cells are known to be dependent on thiamine uptake as genomically encoded genes for the biosynthesis of thiamine are mutated and not active.

For the β-galactosidase assay 5 ml pre-cultures of DH5aZ' cells were prepared in LB Lennox standard medium (lysogeny broth Lennox, 10 g/l tryptone, 5 g/l yeast extract, 5 g/l NaCl in water) and incubated over night at 37°C and 155 rpm. After incubation the optical density was measured photometrically at 600 nm (OD₆₀₀) and the cells were diluted to an optical density of 0.5.

These pre-cultures were used to inoculate β-galactosidase expression cultures at a ratio of 1:500 in a final volume of 8 ml in M9 medium (5x M9 medium containing 15 g/l KH₂PO₄, 5 g/l NH₄Cl, 2.5 g/l NaCl, 30 g/l Na₂HPO₄, in water) containing 40 µl 1M MgSO₄, 80 µl 20 wt% glucose, 75 µl 20µg/µl Casamino Acids (Difco), and 8 µl 100mg/ml Ampicillin, The expression cultures contained either 20 µM of thiamine (Sigma), 500 µM pyrithiamine (Sigma) or 500µM of the compounds according to formulas (1), (2), (3), 4), and (13), respectively. Controls received neither thiamine or pyrithiamine, nor compounds. Samples were run in duplicate and repeated at least three times.

The cultures were incubated for 24 h at 37°C and 150 rpm. After the 24h incubation the optical density was measured. The cells were centrifuged at 4500 g for 5 min and the pellets were washed twice in 800 µl 1x PBS (137mM NaCl, 2.7mM KCl, 10mM Na₂HPO₄x2H₂O, 2mM KH₂PO₄, pH 7.4). The pellets were resuspended in 200 µl lx lysis buffer (Reporter Lysis Buffer, Promega), incubated for 15 min at room temperature (20±2°C), and the cells were pelleted. The supernatants were used for following steps. In duplicate 75 µl of the respective lysates were mixed with 75 µl of 2x assay buffer (200 mM sodium phosphate buffer pH 7.3, 2 µM MgCl₂, 100 mM ß-Mercaptoethanol, 1.33 mg/ml ONPG) and incubated for 5-15 min at 37°C. The reaction was stopped by the addition of 250µl 1M Na₂CO₃ solution and absorbance at 420 nm was measured immediately using a Nanaquant (infinite 200, Tecan). The β-galactosidase reporter gene assay was performed in clear, flat bottom 96-well plates. Miller units were calculated according to the following formula: Miller units = (1000*OD420)/(OD 600*inc.time*culturevol[L]) and Miller units of controls without thiamine or compound added were set to 100 %. Samples were run in duplicate and repeated at least three times.

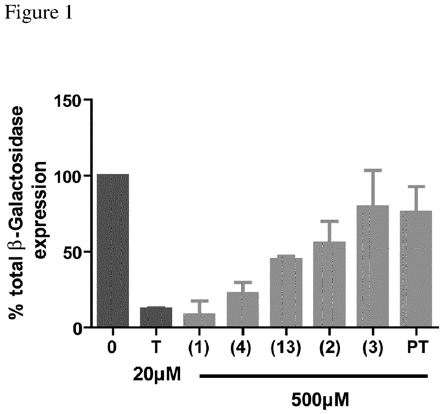

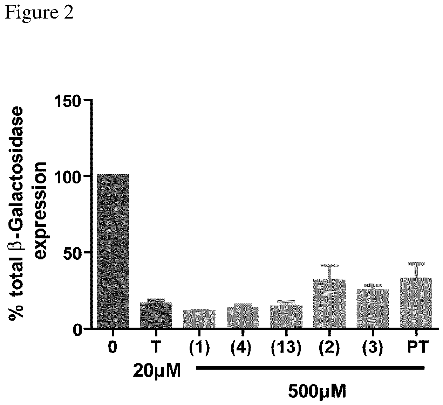

Figure 1 shows the expression of β-galactosidase in the presence of 20µM of thiamine (T), or 500µM of the compounds according to formulas (1), (4), (13), (2), and (3), respectively, pyrithiamine (PT) and an untreated control (0) in *E*. *coli* DH5aZ' cells. Figure 2 shows the expression of β-galactosidase in the presence of 20µM of thiamine (T), or 500µM of the compounds according to formulas (1), (4), (13), (2), and (3), respectively, pyrithiamine (PT) and an untreated control (0) in *E*. *coli* MG1655 cells. As can be seen in Figure 1, the compounds decreased the expression level of β-galactosidase caused by an activation of the thiM riboswitch in DH5αZ'cells. Especially the compound according to formula (1) decreased the expression level of β-galactosidase dramatically.

These experiments show that the compounds according to formulas (1), (2), (3), (4), and (13) can activate the thiM riboswitch. Especially the compound according to formula (1) is able to activate the metabolite-dependent thiM riboswitch.

### Example 19

Investigation of in vivo effects of the compounds according to formulas (1), (2), (3), (4), and (13) by the β-galactosidase reportergene assay in E. coli MG1655 cells

A β-galactosidase reportergene assay using the compounds according to formulas (1), (2), (3), (4), and (13) as described in example 18 also was performed in E. coli MG1655 [pRS414.2thiM] cells (Coli Genetic Stock Center, (CGSC), New Haven, CT, USA) which is a laboratory K12 strain that has 2 mutations (F-, λ⁻, rph-1) in its genome.

The expression cultures contained either 20 µM of thiamine (Sigma), 500 µM pyrithiamine (Sigma) or 500µM of the compounds according to formulas (1), (2), (3), (4), and (13), respectively. Controls received neither thiamine or pyrithiamine, nor compounds. Samples were run in duplicate and repeated at least three times.

Figure 2 shows the expression of β-galactosidase in the presence of 20µM of thiamine (T), or 500µM of the compounds according to formulas (1), (4), (13), (2), and (3), respectively, pyrithiamine (PT) and an untreated control (0) in *E*. *coli* MG1655 cells. As can be seen in Figure 2, the compounds decreased the expression level of β-galactosidase caused by an activation of the thiM riboswitch in MG1655 cells. Especially the compound according to formula (1), (4), and (13) decreased the expression level of β-galactosidase.

Whereas pyrithiamine only shows an effect on gene expression in MG1655 cells, the triazole compounds especially the ones according to formula (1) and (4) caused dramatic riboswitch activation. These experiments show that the compounds according to formulas (1), (2), (3), (4), and (13) can activate the thiM riboswitch.

### Example 20

### Investigation of in vivo effects of the compounds according to formulas (5), (6), (7), (8), (9), (10), (11), and (12) by the β-galactosidase reportergene assay in E. coli DH5aZ' cells

A β-galactosidase reportergene assay using the compounds according to formulas (5), (6), (7), (8), (9), (10), (11), and (12) was performed in E. coli DH5aZ' [pRS414.2thiM] cells as described in example 18.

The expression cultures contained either 2 µM of thiamine (Sigma), or 500 µM of the compounds according to formulas (5), (6), (8), (9), (12), and the trifluoroacetate of the compound according to formula (7), (10), and (11), respectively. Controls received neither thiamine nor compounds. Samples were run in duplicate and repeated at least three times.

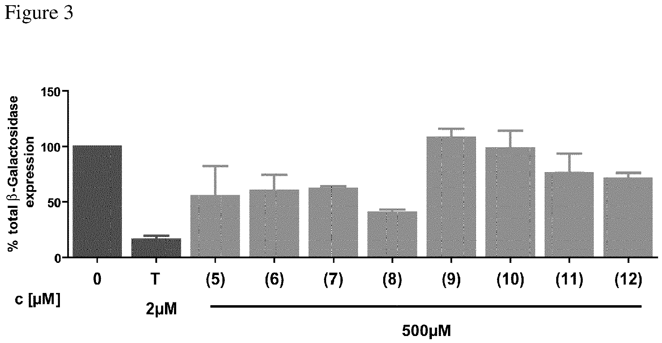

Figure 3 shows the effect on β-galactosidase expression exhibited by thiamine (T) as a positive control and the compounds according to formulas (5), (6), the trifluoroacetate of the compound according to formula (7), compound (8), compound (9), the trifluoroacetates of the compounds according to formulas (10) and (11), respectively, and compound (12) and an untreated control (0) in *E*. *coli* DH5aZ' cells. As can be seen in Figure 3, the compounds decreased the expression level of β-galactosidase, especially the compound according to formula (5), (6), (7) and (8).

This indicates that the compounds according to the invention could be useful antibiotics, when targeting thi-box riboswitches controlling essential genes.

## Claims

1. A compound according to general formula (I) as given as follows and/or racemates, enantiomers, diastereomers, solvates, hydrates, and pharmaceutically acceptable salts and/or esters thereof: 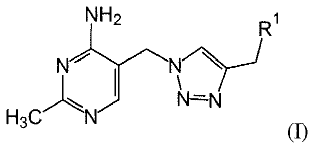 wherein:
R¹ is selected from the group comprising -OH, -CH₂-NH₂, -(CH₂)₂-OH, -(CH₂)₃-OH, -CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given as follows:

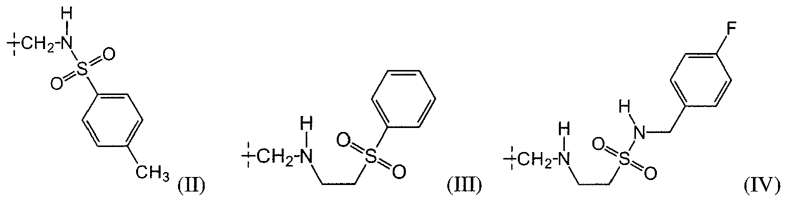

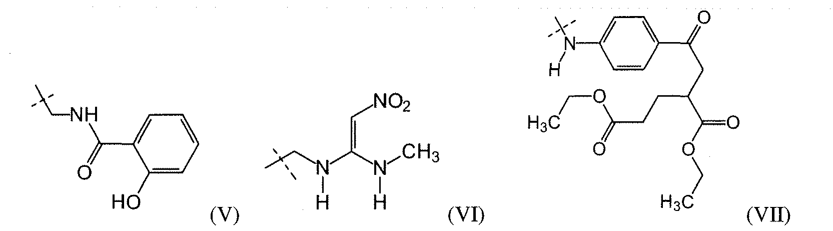

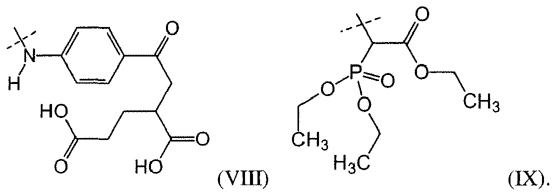

2. The compound according to claim 1, **characterized in that** the compound has the general formula (X) as given as follows and/or racemates, enantiomers, diastereomers, solvates, hydrates, and pharmaceutically acceptable salts and/or esters thereof: 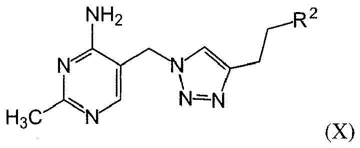 wherein:
R² is selected from the group comprising -NH₂, -CH₂-OH, -(CH₂)₂-OH, -O-S(O)₂CH₃, -NH-S(O)₂CH₃, -NH-C(O)-C(O)-CH₃, -NH-C(O)-CH₂-C(O)-O-CH₂-CH₃, and/or sulfones according to formulas (XI), (XII), and (XIII) as given as follows

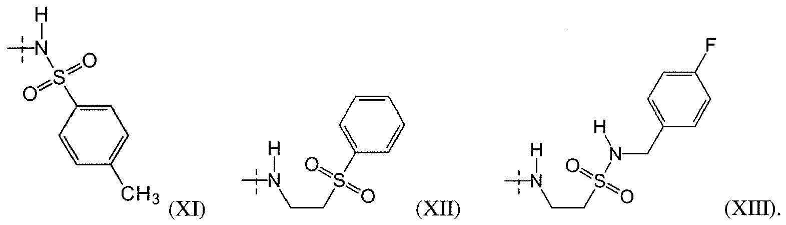

3. The compound according to general formula (I) as given as follows and/or racemates, enantiomers, diastereomers, solvates, hydrates, and pharmaceutically acceptable salts and/or esters thereof: 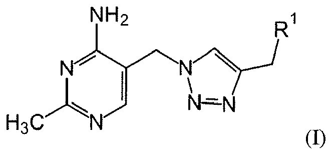 wherein:
R¹ is selected from the group comprising -OH, -CH₂-NH₂, -(CH₂)₂-OH, -(CH₂)₃-OH, -CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given as follows: 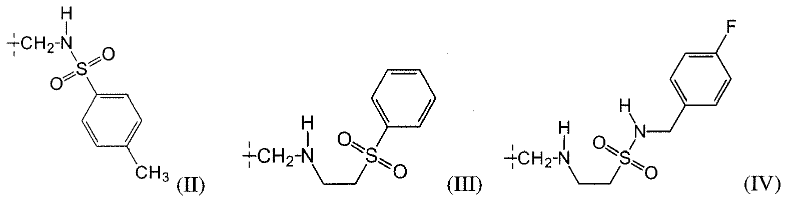 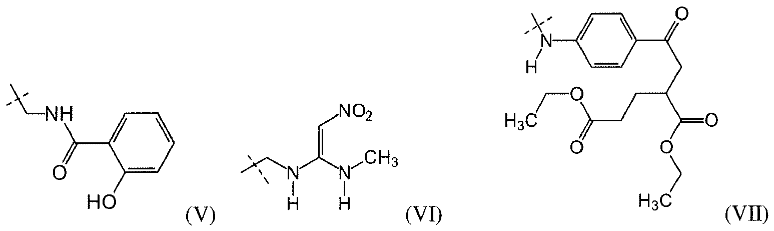 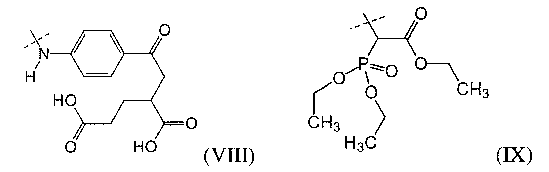
for use as a medicament.

4. The compound according to general formula (I) as given as follows and/or racemates, enantiomers, diastereomers, solvates, hydrates, and pharmaceutically acceptable salts and/or esters thereof: 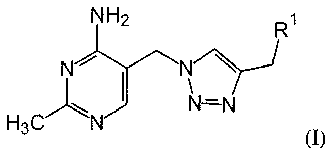 wherein:
R¹ is selected from the group comprising -CH₂-OH, -OH, -CH₂-NH₂, -(CH₂)₂-OH, -(CH₂)₃-OH, - CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given as follows: 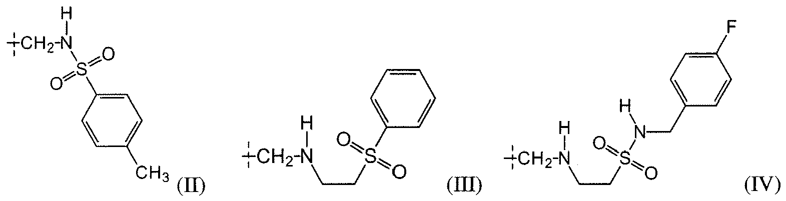 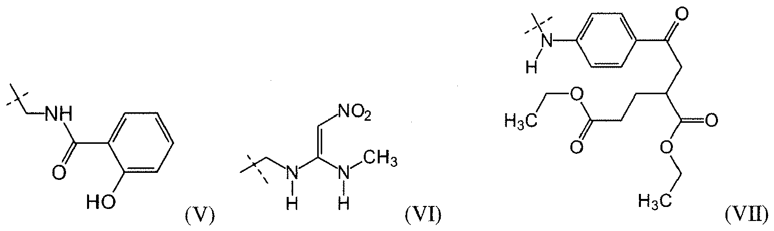 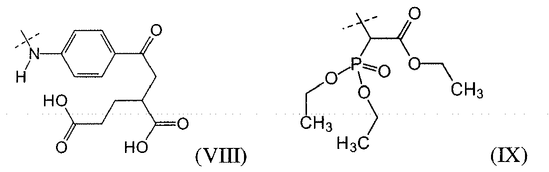
for use in the therapeutic and/or prophylactic treatment of a bacterial infection, particularly for use as an antibiotic.

5. The compound according to claim 4, **characterized in that** the compound has the general formula (X) as given as follows and/or racemates, enantiomers, diastereomers, solvates, hydrates, and pharmaceutically acceptable salts and/or esters thereof: 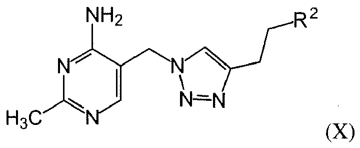 wherein:
R² is selected from the group comprising -OH, -NH₂, -CH₂-OH, -(CH₂)₂-OH, -O-S(O)₂CH₃, -NH-S(O)₂CH₃, -NH-C(O)-C(O)-CH₃, -NH-C(O)-CH₂-C(O)-O-CH₂-CH₃, and/or sulfones according to formulas (XI), (XII), and (XIII) as given as follows

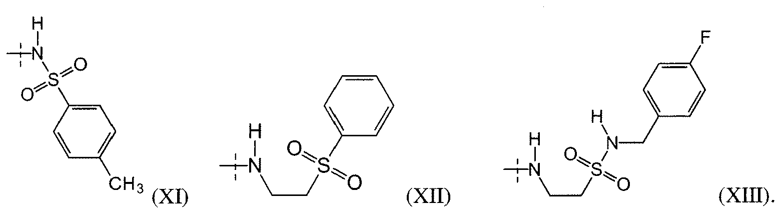

6. The compound according to claims 4 or 5, **characterized in that** the compound is the compound according to formula (1) as indicated below and/or racemates, enantiomers, diastereomers, solvates, hydrates, and pharmaceutically acceptable salts and/or esters thereof:

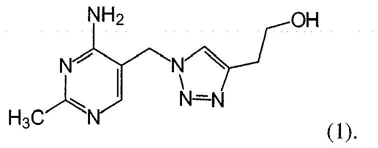

7. A pharmaceutical composition comprising as an active ingredient a compound according to general formula (I) as given as follows and/or racemates, enantiomers, diastereomers, solvates, hydrates, and pharmaceutically acceptable salts and/or esters thereof: 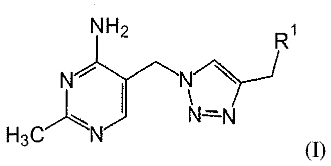 wherein:
R¹ is selected from the group comprising -OH, -CH₂-NH₂, -(CH₂)₂-OH, -(CH₂)₃-OH, -CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given as follows:

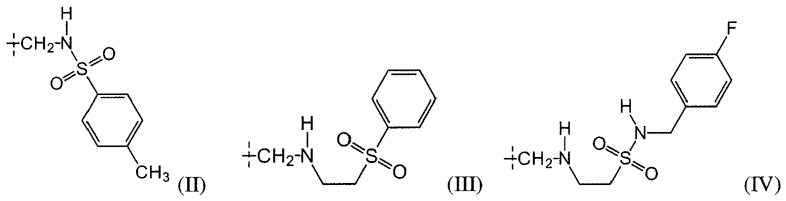

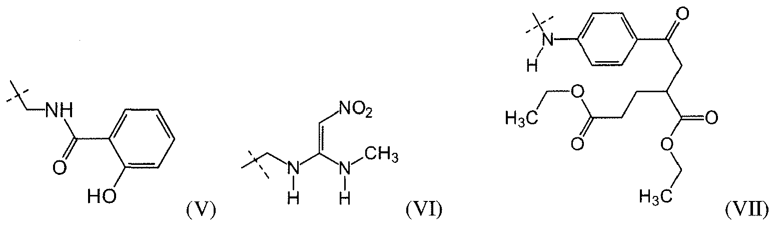

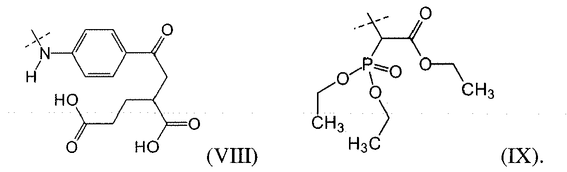

8. A pharmaceutical composition comprising as an active ingredient a compound according to general formula (I) as given as follows and/or racemates, enantiomers, diastereomers, solvates, hydrates, and pharmaceutically acceptable salts and/or esters thereof: 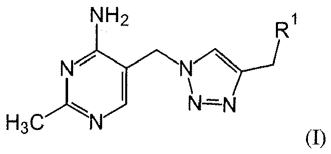 wherein:
R¹ is selected from the group comprising -CH₂-OH, -OH, -CH₂-NH₂, -(CH₂)₂-OH, -(CH₂)₃-OH, - CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given as follows: 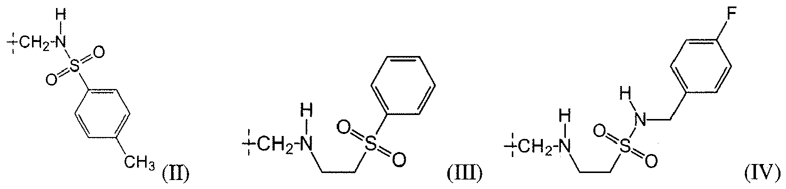 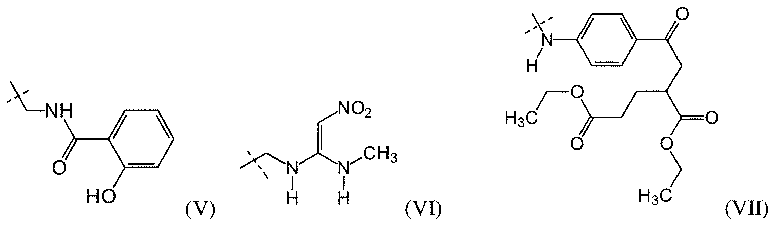 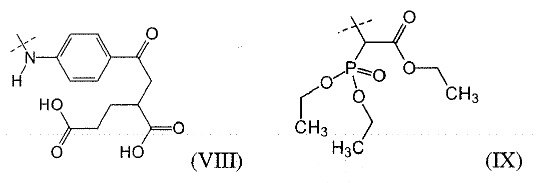
for use in the therapeutic and/or prophylactic treatment of a bacterial infection, particularly for use as an antibiotic.

9. Use of a compound according to general formula (I) as given as follows and/or racemates, enantiomers, diastereomers, solvates, hydrates, and pharmaceutically acceptable salts and/or esters thereof: 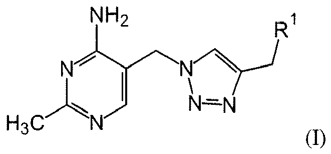 wherein:
R¹ is selected from the group comprising -OH, -CH₂-NH₂, -(CH₂)₂-OH, -(CH₂)₃-OH, -CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given as follows: 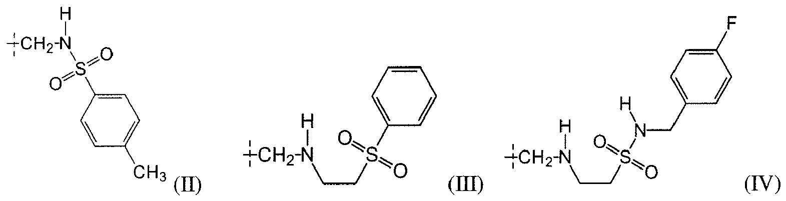 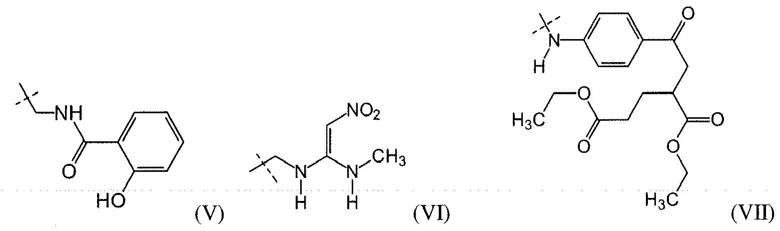 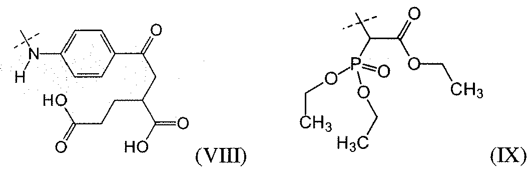
for the manufacture of a medicament.

10. Use of a compound according to general formula (I) as given as follows and/or racemates, enantiomers, diastereomers, solvates, hydrates, and pharmaceutically acceptable salts and/or esters thereof: 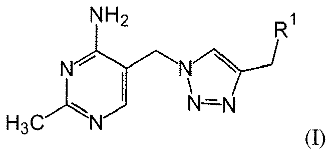 wherein:
R¹ is selected from the group comprising -CH₂-OH, -OH, -CH₂-NH₂, -(CH₂)₂-OH, -(CH₂)₃-OH, - CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given as follows: 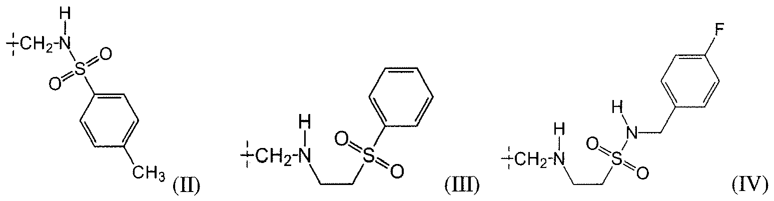 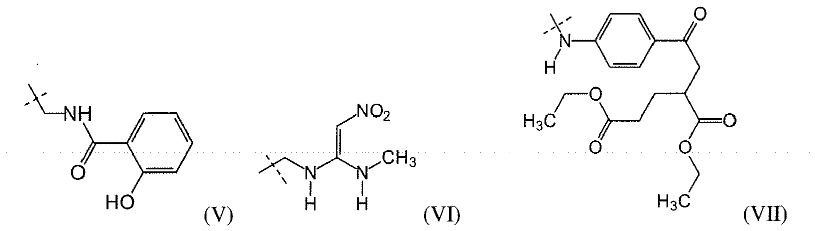 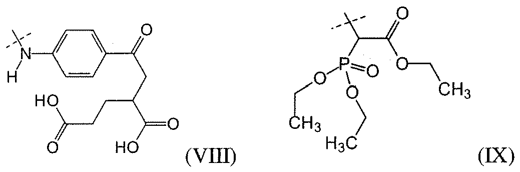
for the manufacture of a medicament for the therapeutic and/or prophylactic treatment of a bacterial infection, particularly for the manufacture of an antibiotic.

11. Method for preparing a compound according to any one of the foregoing claims comprising the steps of:
a) Providing an azide functionalised pyrimidine according to formula (XIV) 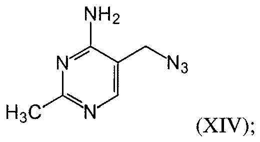
b1) Reacting the azide functionalised pyrimidine according to formula (XIV) with an alkyne according to formula (XV) 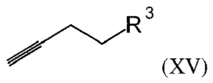 wherein R³ is selected from the group comprising -OH, -NH₂, -CH₂-OH, -(CH₂)₂-OH, and -O-S(O)₂CH₃ to a compound according to formula (XVI) 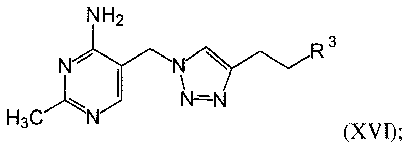 and
c1) Reacting the compound according to formula (XVI) wherein R³ is -NH₂ of step b1) with a compound Cl-SO₂-R⁴ wherein R⁴ is selected from the group comprising -CH₃ or -C₆H₄-CH₃ to a compound according to formula (XVII) 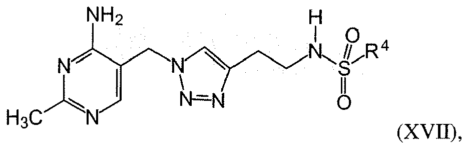 or
c2) Reacting the compound according to formula (XVI) wherein R³ is -NH₂ of step b1) with a compound HOOC-R⁵ wherein R⁵ is selected from the group comprising -CHO, -CH₂-COOC₂H₅ or -C₆H₄-OH to a compound according to formula (XVIII) 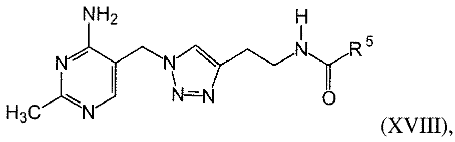 or
c3) Reacting the compound according to formula (XVI) wherein R³ is -NH₂ of step b1) with a compound H₂C=CH-R⁶ to a compound according to formula (XIX) 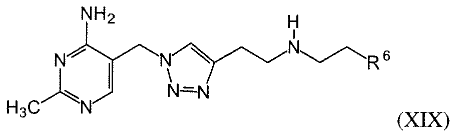 wherein R⁶ is selected from the group comprising structural elements according to formulas (XX), and (XXI) as given as follows 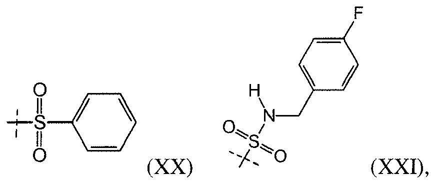 or
c4) Reacting the compound according to formula (XVI) wherein R³ is -NH₂ of step b1) with the compound C(S-CH₃)(NH-CH₃)CH-NO₂ to the compound according to formula (14) 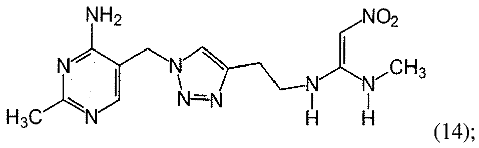 or
b2) Reacting the azide functionalised pyrimidine according to formula (XIV) with an alkyne according to formula (XXII) 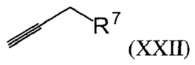 to a compound according to formula (XXIII) 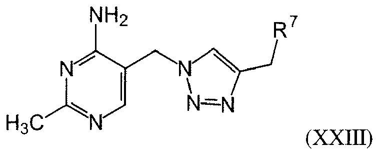 wherein R⁷ is -OH or selected from the group comprising structural elements according to formulas (VII) or (IX) as given as follows

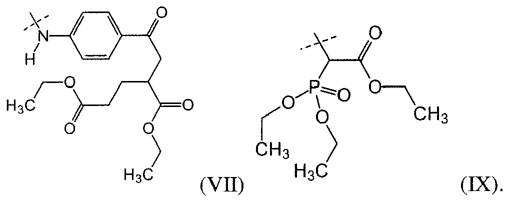

12. A method of treating a bacterial infection, the method comprising administering to the subject a therapeutically effective amount of a compound according to the general formula (I) as given as follows and/or racemates, enantiomers, diastereomers, solvates, hydrates, and pharmaceutically acceptable salts and/or esters thereof: 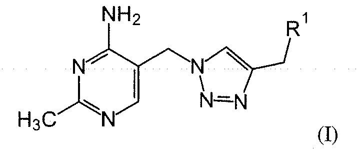 wherein:
R¹ is selected from the group comprising -CH₂-OH, -OH, -CH₂-NH₂, -(CH₂)₂-OH, -(CH₂)₃-OH, - CH₂-O-S(O)₂CH₃, -CH₂-NH-S(O)₂CH₃, -CH₂-NH-C(O)-C(O)-CH₃, -CH₂-NH-C(O)-CH₂-C(O)-O-CH₂-CH₃, and/or structural elements according to formulas (II) to (IX) as given as follows:
